(19) **Europäisches Patentamt / European Patent Office / Office européen des brevets**

(11) **EP 4 433 010 B1**

(12) **EUROPEAN PATENT SPECIFICATION**

(45) Date of publication and mention of the grant of the patent:
**20.08.2025 Bulletin 2025/34**

(21) Application number: **22809324.1**

(22) Date of filing: **11.11.2022**

(51) International Patent Classification (IPC):
**A61F 13/15** *(2006.01)* **A61F 13/49** *(2006.01)*

(52) Cooperative Patent Classification (CPC):
**A61F 13/15739; A61F 13/15699; A61F 13/49011;** A61F 2013/49025; A61F 2013/49033; A61F 2013/49036

(86) International application number:
**PCT/CN2022/131361**

(87) International publication number:
**WO 2023/088179 (25.05.2023 Gazette 2023/21)**

(54) **ABSORBENT ARTICLE WITH FRONT AND/OR BACK WAIST REGIONS HAVING A HIGH-STRETCH ZONE AND A LOW-STRETCH ZONE AND METHODS FOR MAKING**

ABSORBIERENDER ARTIKEL MIT VORDEREN UND/ODER HINTEREN TAILLENBEREICHEN MIT EINER HOCHDEHNZONE UND EINER NIEDERDEHNZONE SOWIE VERFAHREN ZUR HERSTELLUNG

ARTICLE ABSORBANT AYANT DES RÉGIONS DE TAILLE AVANT ET/OU ARRIÈRE AYANT UNE ZONE À ÉTIREMENT ÉLEVÉ ET UNE ZONE À FAIBLE ÉTIREMENT ET PROCÉDÉS DE FABRICATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **19.11.2021 US 202163281113 P**

(43) Date of publication of application:
**25.09.2024 Bulletin 2024/39**

(73) Proprietor: **The Procter & Gamble Company**
**Cincinnati, OH 45202 (US)**

(72) Inventors:
• **LONG, Michael Devin**
  **Ohio 45202 (US)**
• **ROSIAK, Jeffry**
  **Ohio 45202 (US)**
• **BROWN, Tina**
  **Ohio 45202 (US)**
• **GITTINGS, Matthew Alexander**
  **Ohio 45202 (US)**

• **ISHIHARA, Kaoru**
  **Ohio 45202 (US)**
• **OGAWA, Kyzuya**
  **Hyogo 6740093 (JP)**
• **ZHOU, Wei**
  **Guangdong 510555 (CN)**
• **HUANG, Zhiqiang**
  **Guangdong 510555 (CN)**
• **ZHU, Tianzhuo**
  **Beijing 101312 (CN)**
• **TONG, Ling**
  **Beijing 101312 (CN)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-2021/126150     US-A1- 2018 168 880**
**US-A1- 2021 275 364     US-B2- 10 398 607**

**Description**

FIELD OG THE INVENTION

The present disclosure relates to absorbent articles, and more particularly, to absorbent articles having front and/or back waist regions including high-stretch and low-stretch zones.

BACKGROUND OF THE INVENTION

Along an assembly line, various types of articles, such as for example, diapers and other absorbent articles, may be assembled by adding components to and/or otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. In some cases, individual components created from an advancing web or webs are combined with other individual components created from other advancing webs. Webs of material and component parts used to manufacture diapers may include: backsheets, topsheets, leg cuffs, waist bands, absorbent core components, front and/or back ears, fastening components, and various types of elastic webs and components such as front and/or back waist panels, leg elastics, barrier leg cuff elastics, stretch side panels, and waist elastics. Once the desired component parts are assembled, the advancing web(s) and component parts are subjected to a final knife cut to separate the web(s) into discrete diapers or other absorbent articles.

Some absorbent articles have components that include elastomeric laminates. Such elastomeric laminates may include an elastic material bonded to one or more nonwovens. The elastic material may include an elastic film and/or elastic strands. In some laminates, a plurality of elastic strands are joined to a nonwoven while the plurality of strands are in a stretched condition so that when the elastic strands relax, the nonwoven gathers in the locations where the nonwoven is bonded to the elastic strands, and in turn, forms corrugations and rugosities. The resulting elastomeric laminate is stretchable to the extent that the corrugations allow the elastic strands to elongate.

Some absorbent articles in the form of diaper pants are configured with an absorbent chassis connected with front and back elastic belts, wherein opposing end regions of the front and back belts are connected with each other at side seams. In some instances, the elasticity of the front and back belts is removed in regions where the chassis connects with the belts. Thus, in some converting configurations adapted to assemble such diaper pants, stretched elastic strands are glued between two continuous nonwoven webs to form an elastic laminate. Regions of the elastic strands may then be intermittently deactivated along the length of the elastic laminate by cutting the elastic strands. Subsequent to deactivating the elastic strands, the elastic laminate may be subjected to additional handling and converting operations. WO2021/126150A1 discloses such an absorbent article comprising a non-elastomeric region where the elastic material was deactivated.

In some operations, an elastic laminate may advance through a cutting station that cuts the elastic in the advancing laminate. For example, the elastic strands may be cut with a knife blade configured with a relatively smooth and/or radiused edge that creates enough compressive load to rupture the elastic strands without cutting through the nonwoven webs. However, when attempting to cut elastic strands with relatively low decitex, it can be challenging to utilize such strand cutting technology to successfully and consistently cut such elastic strands without damaging the nonwovens. As such, consistently cutting the relatively low decitex elastic strands may require a process that cuts through the nonwoven webs, which in turn, may damage the elastic laminate, resulting in a relatively poor aesthetic appearance. In addition, the ends of the cut elastic stands may snap back and in an uncontrolled fashion and consequently may end up in undesired locations within the laminate. Further, cutting the nonwoven webs while deactivating the elastics in an elastic laminate may weaken the laminate, making the laminate relatively more likely to tear, and/or may otherwise result in control and handling difficulties associated with differential stretch characteristics within the laminate.

Consequently, it would be beneficial to provide methods and apparatuses that are configured to cut relatively low decitex elastic strands in elastic laminates in such a way to reduce web control and handling difficulties while helping to increase the aesthetic appearance of the laminate when utilized in an assembled product.

SUMMARY OF THE INVENTION

The absorbent article of the invention comprises: a body facing surface and a garment facing surface; a front waist region and a back waist region, the back waist region separated from the front waist region by a crotch region, the front waist region comprising a front waist edge, and the back waist region comprising a back waist edge, wherein a longitudinal axis extends perpendicularly through the front waist edge and the back waist edge, and wherein a lateral axis extends perpendicularly to the longitudinal axis; an absorbent assembly extending longitudinally through the crotch region between the front waist region and the back waist region, the absorbent assembly positioned between the body facing surface and the garment facing surface; wherein at least one of the front waist region and the back waist region comprises: an elastic material positioned between and connected with a first substrate and a second substrate; a first high-stretch zone and a second high-stretch zone separated laterally by a low-stretch zone, wherein the first and second high-stretch zones are elasticated by the elastic material; wherein the low-stretch zone comprises cut lines separating the elastic material into first discrete pieces and second discrete pieces; wherein the first discrete pieces of elastic material comprise a first length and wherein the second discrete pieces of elastic material comprise a second length, wherein the second length is greater than the first length; and wherein each cut line is oriented to define an offset angle relative to the lateral axis that is greater than 0 degrees and less than 45 degrees.

BRIEF DESCRIPTION OF THE DRAWINGS

Figure 1 shows a perspective views of a diaper pant in a pre-fastened configuration.
Figure 2A shows a plan view of a diaper pant with the portion of the diaper that faces away from a wearer oriented toward the viewer.
Figure 2B shows a plan view of a diaper pant with the portion of the diaper that faces toward a wearer oriented toward the viewer.
Figure 2C shows a plan view of a diaper pant with the portion of the diaper that faces away from a wearer oriented toward the viewer, illustrating first and second belt size and shape features.
Figure 2D shows a plan view of a diaper pant with the portion of the diaper that faces away from a wearer oriented toward the viewer, illustrating first and second belt size and shape features.
Figure 2E shows a plan view of a diaper pant with the portion of the diaper that faces away from a wearer oriented toward the viewer, illustrating first and second belt size and shape features.
Figure 3 is a cross-sectional view of the diaper pant of Figure 2A taken along line 3-3 showing first and second elastic belts provided with panel layers.
Figure 4A is a detailed view of a portion of a low-stretch zone showing details of cut lines therein.
Figure 4B is a detailed view of a portion of a low-stretch zone showing details of cut lines and discrete elastic pieces therein.
Figure 5 is a schematic side view of a converting apparatus adapted to manufacture an elastomeric laminate including a plurality of elastic strands positioned between a first substrate and a second substrate.
Figure 6 is a view of the converting apparatus of Figure 5 taken along line 6-6.
Figure 7 is a schematic side view of a converting apparatus adapted to create low-stretch zones in an elastomeric laminate.
Figure 8 is a view of the converting apparatus of Figure 7 taken along line 8-8.
Figure 9 is a detailed view of an elastic cutting apparatus showing details of blades thereon.
Figure 10 is a schematic view of a diaper pant assembly process.
Figure 11 is a view of an advancing elastomeric laminate advancing from a cutting device to a slitter device.
Figure 11A is a view of an advancing elastomeric laminate advancing from a slitter device to a cutting device.
Figure 12A is a view of an advancing elastomeric laminate having a first width advancing from a cutting device.
Figure 12B is a view of an advancing elastomeric laminate having a second width advancing from a cutting device.
Figure 12C is a schematic view of another embodiment of an elastic laminate showing details of cut lines.
Figure 12D is an enlarged view of Figure 12C.
Figure 12E is a cross sectional view of a diaper pant taken along the longitudinal axis.

DETAILED DESCRIPTION OF THE INVENTION

DEFINITIONS

The following term explanations may be useful in understanding the present disclosure:
"Absorbent article" refers to devices, which absorb and contain body exudates and, more specifically, refers to devices, which are placed against or in proximity to the body of the wearer to absorb and contain the various exudates discharged from the body. Exemplary absorbent articles include diapers, training pants, pull-on pant-type diapers (i.e., a diaper having a pre-formed waist opening and leg openings such as illustrated in U.S. Pat. No. 6,120,487), refastenable diapers or pant-type diapers, incontinence briefs and undergarments, diaper holders and liners, feminine hygiene garments such as panty liners, absorbent inserts, menstrual pads and the like.
"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

The terms "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force. Elastomeric materials may include elastomeric films, scrims, nonwovens, ribbons, strands and other sheet-like structures.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein, the term "distal" is used to describe a position situated away from a center of a body or from a point of attachment, and the term "proximal" is used to describe a position situated nearer to a center of a body or a point of attachment.

The term "substrate" is used herein to describe a material which is primarily two-dimensional (i.e., in an XY plane) and whose thickness (in a Z direction) is relatively small (i.e., 1/10 or less) in comparison to its length (in an X direction) and width (in a Y direction). Nonlimiting examples of substrates include a web, layer or layers or fibrous materials, nonwovens, films and foils such as polymeric films or metallic foils. These materials may be used alone or may comprise two or more layers laminated together. As such, a web is a substrate.

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

"Pre-strain" refers to the strain imposed on an elastic or elastomeric material prior to combining it with another element of the elastomeric laminate or the absorbent article. Pre-strain is determined by the following equation Pre-strain = ((extended length of the elastic-relaxed length of the elastic)/relaxed length of the elastic)*100.

"Decitex" also known as Dtex is a measurement used in the textile industry used for measuring yarns or filaments. 1 Decitex = 1 gram per 10,000 meters. In other words, if 10,000 linear meters of a yarn or filament weights 500 grams that yarn or filament would have a decitex of 500.

The term "taped diaper" (also referred to as "open diaper") refers to disposable absorbent articles having an initial front waist region and an initial back waist region that are not fastened, pre-fastened, or connected to each other as packaged, prior to being applied to the wearer. A taped diaper may be folded about the lateral centerline with the interior of one waist region in surface to surface contact with the interior of the opposing waist region without fastening or joining the waist regions together. Example taped diapers are disclosed in various suitable configurations U.S. Patent Nos. 5,167,897, 5,360,420, 5,599,335, 5,643,588, 5,674,216, 5,702,551, 5,968,025, 6,107,537, 6,118,041, 6,153,209, 6,410,129, 6,426,444, 6,586,652, 6,627,787, 6,617,016, 6,825,393, and 6,861,571; and U.S. Patent Publication Nos. 2013/0072887 A1; 2013/0211356 A1; and 2013/0306226 A1.

The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed or pre-fastened by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, back waist fastened or seamed). Example diaper pants in various configurations are disclosed in U.S. Patent Nos. 4,940,464; 5,092,861; 5,246,433; 5,569,234; 5,897,545; 5,957,908; 6,120,487; 6,120,489; 7,569,039 and U.S. Patent Publication Nos. 2003/0233082 A1; 2005/0107764 A1, 2012/0061016 A1, 2012/0061015 A1; 2013/0255861 A1; 2013/0255862 A1; 2013/0255863 A1; 2013/0255864 A1; and 2013/0255865 A1.

"Closed-form" means opposing waist regions are joined, as packaged, either permanently or refastenably to form a continuous waist opening and leg openings.

"Open-form" means opposing waist regions are not initially joined to form a continuous waist opening and leg openings but comprise a closure means such as a fastening system to join the waist regions to form the waist and leg openings before or during application to a wearer of the article.

The present disclosure relates to absorbent articles including elastic laminates, and more particularly, to absorbent articles having elastic laminates in front and/or back waist regions with high-stretch zones and low-stretch zones. In some configurations, an absorbent article may include a body facing surface and a garment facing surface with a front waist region and a back waist region separated by a crotch region. The front waist region comprises a front waist edge, and the back waist region comprises a back waist edge. A longitudinal axis extends perpendicularly through the front waist edge and the back waist edge, and a lateral axis extends perpendicularly to the longitudinal axis. An absorbent assembly positioned between the body facing surface and the garment facing surface may extend longitudinally through the crotch region between the front waist region and the back waist region. At least one of the front waist region and the back waist region may comprise: an elastic material positioned between and connected with a first substrate and a second substrate, and a first high-stretch zone and a second high-stretch zone separated laterally by a low-stretch zone. The first and second high-stretch zones are elasticated by the elastic material. The low-stretch zone comprises cut lines separating the elastic material into first discrete pieces having a first length and second discrete pieces having a second length, wherein the second length is greater than the first length. In some configurations, each cut line is oriented to define an offset angle relative to the lateral axis that is greater than 0 degrees and less than 45 degrees, specifically reciting all 1 degree increments within the above-recited range and all ranges formed therein or thereby. In some configurations, the cut lines penetrate through the elastic material, the first substrate, and the second substrate. In turn, the offset angles of the cut lines help to prevent the cut lines from opening when lateral forces are applied to the respective first and second substrates. In addition, the cut length differences help to mask the cut lines.

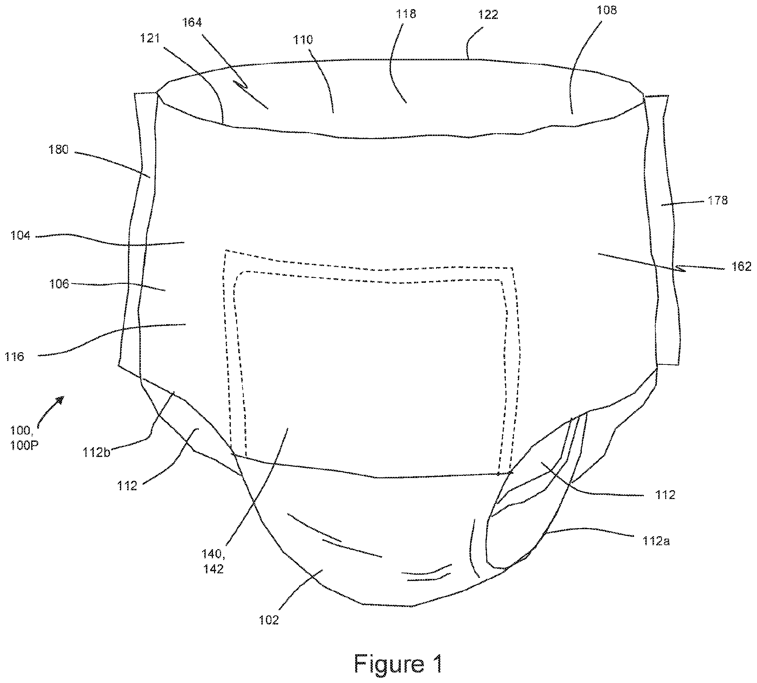

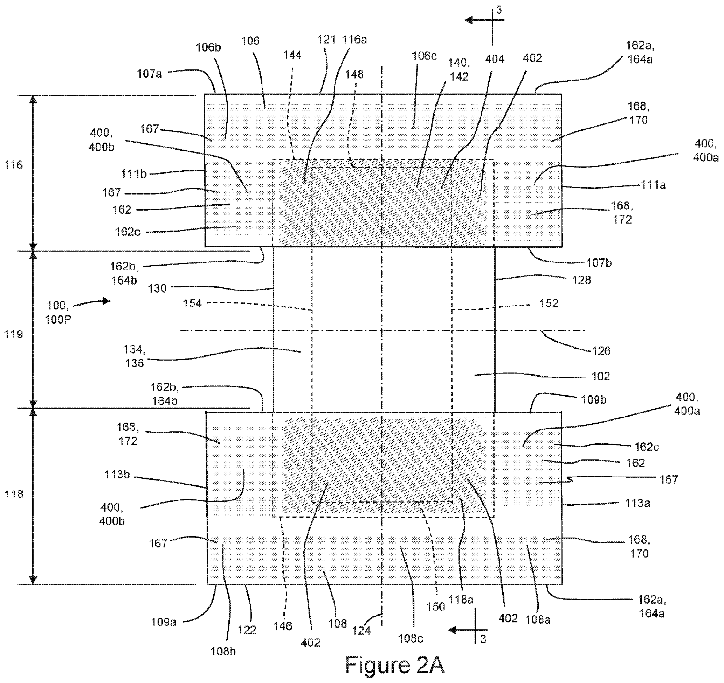

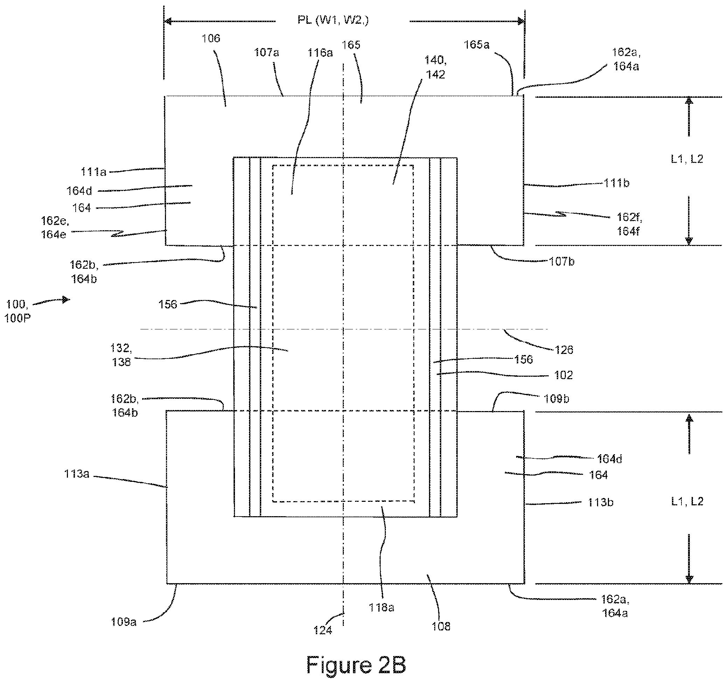

Figures 1-2B show an example of an absorbent article 100 in the form of a diaper pant 100P that may include components constructed from elastic laminates assembled in accordance with the configurations disclosed herein. In particular, Figure 1 shows a perspective views of a diaper pant 100P in a pre-fastened configuration. Figure 2A shows a plan view of the diaper pant 100P with the portion of the diaper that faces away from a wearer oriented toward the viewer, and Figure 2B shows a plan view of the diaper pant 100P with the portion of the diaper that faces toward a wearer oriented toward the viewer. The diaper pant 100P includes a chassis 102 and a ring-like elastic belt 104. As discussed below in more detail, a first elastic belt 106 and a second elastic belt 108 are bonded together to form the ring-like elastic belt 104.

With continued reference to Figures 1-2B, the diaper pant 100P and the chassis 102 each include a first waist region 116, a second waist region 118, and a crotch region 119 disposed intermediate the first and second waist regions. It may also be described that the chassis 102 includes a first end region 116a, a second end region 118a, and a crotch region 119 disposed intermediate the first and second end regions 116a, 118a. The first waist region 116 may be configured as a front waist region, and the second waist region 118 may be configured as back waist region. The diaper 100P may also include a laterally extending front waist edge 121 in the front waist region 116 and a longitudinally opposing and laterally extending back waist edge 122 in the back waist region 118. To provide a frame of reference for the present discussion, the diaper 100P and chassis 102 of Figures 2A and 2B are shown with a longitudinal axis 124 and a lateral axis 126. In some embodiments, the longitudinal axis 124 may extend through the front waist edge 121 and through the back waist edge 122. And the lateral axis 126 may extend through a first longitudinal or right side edge 128 and through a second longitudinal or left side edge 130 of the chassis 102. As previously mentioned, the longitudinal axis 124 extends perpendicularly through the front waist edge 121 and the back waist edge 122, and the lateral axis 126 extends perpendicularly to the longitudinal axis 124.

As shown in Figures 1-2B, the diaper pant 100P may include an inner, body facing surface 132, and an outer, garment facing surface 134. The chassis 102 may include a backsheet 136 and a topsheet 138. The chassis 102 may also include an absorbent assembly 140, including an absorbent core 142, disposed between a portion of the topsheet 138 and the backsheet 136. As discussed in more detail below, the diaper 100P may also include other features, such as leg elastics and/or leg cuffs to enhance the fit around the legs of the wearer.

As shown in Figure 2A, the periphery of the chassis 102 may be defined by the first longitudinal side edge 128, a second longitudinal side edge 130, a first laterally extending end edge 144 disposed in the first waist region 116, and a second laterally extending end edge 146 disposed in the second waist region 118. Both side edges 128 and 130 extend longitudinally between the first end edge 144 and the second end edge 146. As shown in Figure 2A, the laterally extending end edges 144 and 146 are located longitudinally inward from the laterally extending front waist edge 121 in the front waist region 116 and the laterally extending back waist edge 122 in the back waist region 118. When the diaper pant 100P is worn on the lower torso of a wearer, the front waist edge 121 and the back waist edge 122 may encircle a portion of the waist of the wearer. At the same time, the side edges 128 and 130 may encircle at least a portion of the legs of the wearer. And the crotch region 119 may be generally positioned between the legs of the wearer with the absorbent core 142 extending from the front waist region 116 through the crotch region 119 to the back waist region 118.

As previously mentioned, the diaper pant 100P may include a backsheet 136. The backsheet 136 may also define the outer, garment facing surface 134 of the chassis 102. The backsheet 136 may also comprise a woven or nonwoven material, polymeric films such as thermoplastic films of polyethylene or polypropylene, and/or a multi-layer or composite materials comprising a film and a nonwoven material. The backsheet may also comprise an elastomeric film. An example backsheet 136 may be a polyethylene film having a thickness of from about 0.012 mm (0.5 mils) to about 0.051 mm (2.0 mils). Further, the backsheet 136 may permit vapors to escape from the absorbent core (i.e., the backsheet is breathable) while still preventing exudates from passing through the backsheet 136.

Also described above, the diaper pant 100P may include a topsheet 138. The topsheet 138 may also define all or part of the inner, wearer facing surface 132 of the chassis 102. The topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art. Topsheets 138 may be selected from high loft nonwoven topsheets, apertured film topsheets and apertured nonwoven topsheets. Exemplary apertured films may include those described in U.S. Patent Nos. 5,628,097; 5,916,661; 6,545,197; and 6,107,539.

As mentioned above, the diaper pant 100P may also include an absorbent assembly 140 that is joined to the chassis 102. As shown in Figure 2A, the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the diaper. Exemplary absorbent structures for use as the absorbent core of the present disclosure are described in U.S. Patent Nos. 4,610,678; 4,673,402; 4,888,231; and 4,834,735.

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprise primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Patent Nos. 5,599,335; 5,562,646; 5,669,894; and 6,790,798 as well as U.S. Patent Publication Nos. 2004/0158212 A1 and 2004/0097895 A1.

As previously mentioned, the diaper 100P may also include elasticized leg cuffs 156. It is to be appreciated that the leg cuffs 156 can be and are sometimes also referred to as leg bands, side flaps, barrier cuffs, elastic cuffs or gasketing cuffs. The elasticized leg cuffs 156 may be configured in various ways to help reduce the leakage of body exudates in the leg regions. Example leg cuffs 156 may include those described in U.S. Patent Nos. 3,860,003; 4,909,803; 4,695,278; 4,795,454; 4,704,115; 4,909,803; and U.S. Patent Publication No. 2009/0312730 A1.

As mentioned above, diaper pants may be manufactured with a ring-like elastic belt 104 and provided to consumers in a configuration wherein the front waist region 116 and the back waist region 118 are connected to each other as packaged, prior to being applied to the wearer. As such, diaper pants may have a continuous perimeter waist opening 110 and continuous perimeter leg openings 112 such as shown in Figure 1. The ring-like elastic belt may be formed by joining a first elastic belt to a second elastic belt with a permanent side seam or with an openable and reclosable fastening system disposed at or adjacent the laterally opposing sides of the belts.

As previously mentioned, the ring-like elastic belt 104 may be defined by a first elastic belt 106 connected with a second elastic belt 108. As shown in Figures 2A and 2B, the first elastic belt 106 extends between a first longitudinal side edge 111a and a second longitudinal side edge 111b and defines first and second opposing end regions 106a, 106b and a central region 106c. And the second elastic 108 belt extends between a first longitudinal side edge 113a and a second longitudinal side edge 113b and defines first and second opposing end regions 108a, 108b and a central region 108c. The distance between the first longitudinal side edge 111a and the second longitudinal side edge 111b defines the pitch length, PL, of the first elastic belt 106, and the distance between the first longitudinal side edge 113a and the second longitudinal side edge 113b defines the pitch length, PL, of the second elastic belt 108. The central region 106c of the first elastic belt is connected with the first waist region 116 or first end region 116a of the chassis 102, and the central region 108c of the second elastic belt 108 is connected with the second waist region 118 or second end region 118a of the chassis 102. As shown in Figure 1, the first end region 106a of the first elastic belt 106 is connected with the first end region 108a of the second elastic belt 108 at first side seam 178, and the second end region 106b of the first elastic belt 106 is connected with the second end region 108b of the second elastic belt 108 at second side seam 180 to define the ring-like elastic belt 104 as well as the waist opening 110 and leg openings 112. It is to be appreciated that the first belt 106 and the second belt 108 may be permanently or refastenably connected with each other at the first side seam 178 and the second side seam 180.

As shown in Figures 2A and 2B, the first elastic belt 106 also defines an outer laterally extending edge 107a and an inner laterally extending edge 107b, and the second elastic belt 108 defines an outer laterally extending edge 109a and an inner laterally extending edge 109b. As such, a perimeter edge 112a of one leg opening may be defined by portions of the inner laterally extending edge 107b of the first elastic belt 106, the inner laterally extending edge 109b of the second elastic belt 108, and the first longitudinal or right side edge 128 of the chassis 102. And a perimeter edge 112b of the other leg opening may be defined by portions of the inner laterally extending edge 107b, the inner laterally extending edge 109b, and the second longitudinal or left side edge 130 of the chassis 102. The outer laterally extending edges 107a, 109a may also define the front waist edge 121 and the laterally extending back waist edge 122 of the diaper pant 100P.

It is to be appreciated that the first elastic belt 106 and the second elastic belt 108 may define different sizes and shapes. In some configurations, the first elastic belt 106 and/or second elastic belt 108 may define curved contours. For example, the inner lateral edges 107b, 109b of the first and/or second elastic belts 106, 108 may include non-linear or curved portions in the first and second opposing end regions. Such curved contours may help define desired shapes to leg opening 112, such as for example, relatively rounded leg openings. In addition to having curved contours, the elastic belts 106, 108 may include elastic strands 168 that extend along non-linear or curved paths that may correspond with the curved contours of the inner lateral edges 107b, 109b.

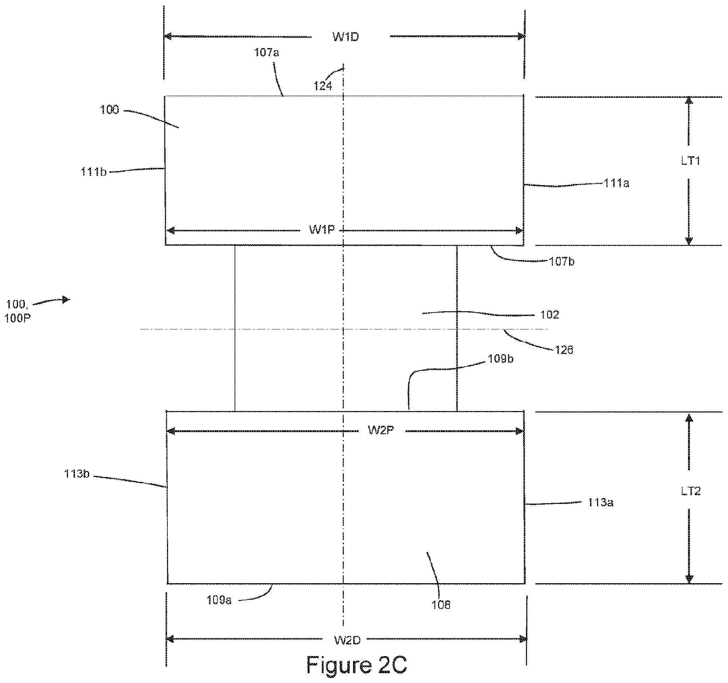

Figure 2C shows a configuration wherein the first elastic belt 106 and the second elastic belt 108 both define generally rectangular shapes. For example, as shown in Figure 2C, the outer laterally extending edge 107a of the first elastic belt 106 may comprise a lateral width of W1D and the inner laterally extending edge 107b may comprise a lateral width of W1P, wherein W1D and W1P are equal or substantially equal. In addition, the outer laterally extending edge 109a of the second elastic belt 108 may comprise a lateral width of W2D and the inner laterally extending edge 109b may comprise a lateral width of W2P, wherein W2D and W2P are equal or substantially equal.

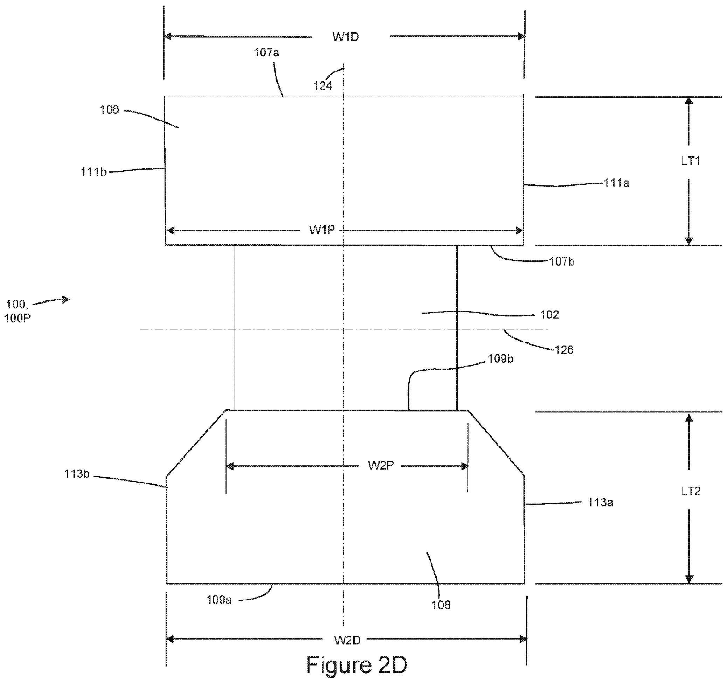

In some configurations, at least one of the first elastic belt 106 and the second elastic belt 108 may comprise lateral edges having different lengths. For example, Figure 2D shows a configuration wherein the first elastic belt 106 defines a generally rectangular shape, such as described with reference to Figure 2C, and wherein the outer laterally extending edge 109a of the second elastic belt 108 and the inner laterally extending edge 109b have different lengths. As shown in Figure 2D, the outer laterally extending edge 109a of the second elastic belt 108 may comprise a lateral width of W2D and the inner laterally extending edge 109b may comprise a lateral width of W2P, wherein W2D is greater than W2P.

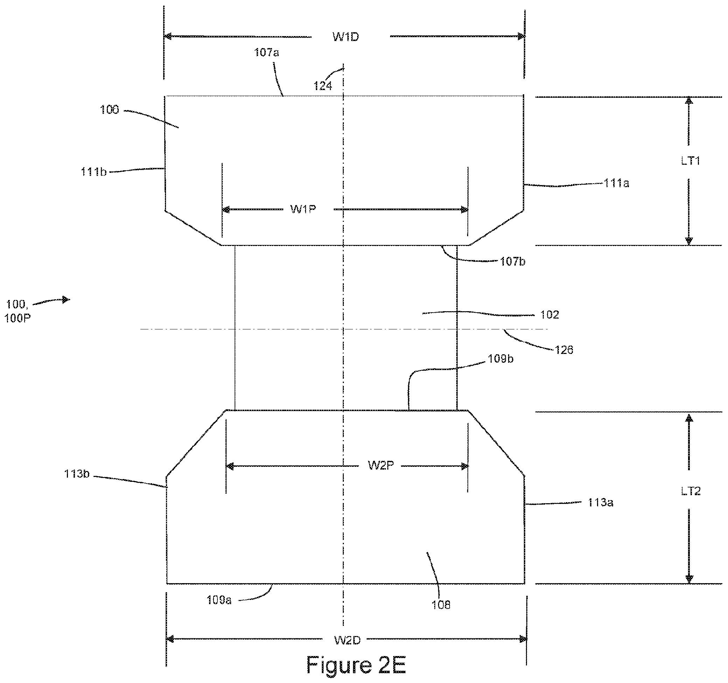

In some configurations, both the first elastic belt 106 and the second elastic belt 108 may comprise lateral edges having different lengths. For example, Figure 2E shows a configuration wherein the outer laterally extending edge 107a of the first elastic belt 106 and the inner laterally extending edge 107b have different lengths, and wherein the outer laterally extending edge 109a of the second elastic belt 108 and the inner laterally extending edge 109b have different lengths. As shown in Figure 2E, the outer laterally extending edge 107a of the first elastic belt 107 may comprise a lateral width of W1D and the inner laterally extending edge 107b may comprise a lateral width of W1P, wherein W1D is greater than W1P, and wherein the outer laterally extending edge 109a of the second elastic belt 108 may comprise a lateral width of W2D and the inner laterally extending edge 109b may comprise a lateral width of W2P, wherein W2D is greater than W2P.

With reference to Figures 2C-2E, the first elastic belt 106 may define a longitudinal length LT1 extending between outer laterally extending edge 107a and the inner laterally extending edge 107b, and the second elastic belt 108 may define a longitudinal length LT2 extending between outer laterally extending edge 109a and the inner laterally extending edge 109b. In some configurations, LT1 may be equal to LT2. In some configurations, LT1 may be less or greater than LT2. With continued reference to Figures 2C-2E, in some configurations, W1D may be equal to W1P, or W1D may be different than W1P. In some configurations, W2D may be equal to W2P, or W2D may be different than W2P. In some configurations, W1D and/or W1P may be equal to or different W2D and/or W2P.

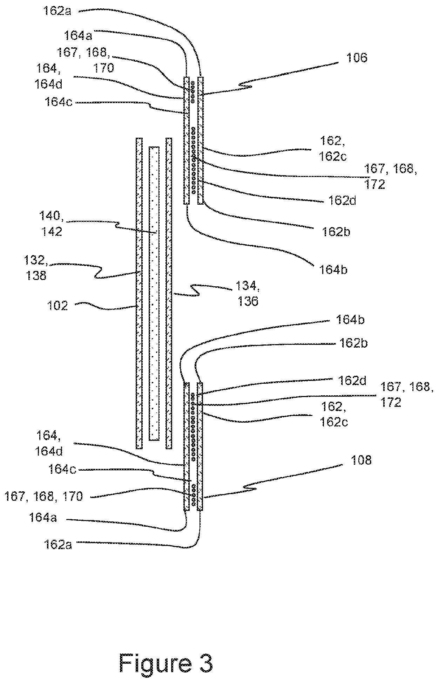

With reference to Figures 2A, 2B, and 3, the first elastic belt 106 and the second elastic belt 108 may also each include a first substrate 162 and a second substrate 164. The first substrates 162 may be oriented to define at least a portion of the garment facing surfaces of the first elastic belt 106 and the second elastic belt 108, and the second substrates 164 may be oriented to define at least a portion of the wearer facing surfaces of the first elastic belt 106 and the second elastic belt 108. The first substrate 162 may extend from a proximal edge 162b to a distal edge 162a for a maximum length L1, and the second substrate 164 may extend from a proximal edge 164b to a distal edge 164a for a maximum length L2. It is to be appreciated that the distal edge 162a and/or the proximal edge 162b of the first substrate 162 may be straight and/or curved and/or may be parallel or unparallel to each other. It is also to be appreciated that the distal edge 164a and/or the proximal edge 164b of the second substrate 164 may be straight and/or curved and/or may be parallel or unparallel to each other. As such, the maximum length L1 refers to the longest distance extending longitudinally between the distal edge 162a and the proximal edge 162b of the first substrate 162, and the maximum length L2 refers to the longest distance extending longitudinally between the distal edge 164a and the proximal edge 164b of the second substrate 164. In some configurations, the distal edge 162a of the first substrate 162 may define at least a portion of the front waist edge 121 and/or at least a portion of back waist edge 122, and/or the distal edge 164a of the second substrate 164 may define at least a portion of the front waist edge 121 and/or at least a portion of back waist edge 122. As such, in some configurations, the distal edge 162a of the first substrate 162 and/or the distal edge 164a of the second substrate 164 may define at least a portion of the waist opening 110. It is also to be appreciated that the first substrate 162 and/or the second substrate 164 may extend continuously from the first belt 106 to the second belt 108.

It is to be appreciated that the first substrate 162 and the second substrate 164 may define various lateral widths that may or may not be equal. For example, as shown in Figure 2B, the first substrate 162 may extend laterally between a first longitudinal edge 162e and a second longitudinal edge 162f to define a first lateral width W1, and the second substrate 164 may extend laterally between a first longitudinal edge 164e and a second longitudinal edge 164f to define a second lateral width W2.

In some configurations, the proximal edge 162b of the first substrate 162 and/or the proximal edge 164b of the second substrate 164 may extend laterally across the backsheet 134. As shown in Figures 2A-3, the first substrate 162 includes a garment facing surface 162c and an opposing wearer facing surface 162d, and the second substrate 164 includes a garment facing surface 164c and an opposing wearer facing surface 164d. In some configurations, the first elastic belt 106 and/or the second elastic belt 108 may include a folded portion of at least the first substrate 162 and/or the second substrate 164. For example, the first elastic belt 106 and/or the second elastic belt 108 may include a folded portion of the first substrate 162 extending longitudinally between a fold line in the first substrate 162 and a lateral edge. As such, the folded portion of the first substrate 162 may be connected with the wearer facing surface 164d of the second substrate 164. In another example, the first elastic belt 106 and/or the second elastic belt 108 may include a folded portion of the second substrate 164 extending longitudinally between a fold line in the second substrate 164 and a lateral edge. As such, the folded portion of the second substrate 164 may be connected with the garment facing surface 162c of the first substrate 162. As such, in some configurations, a fold line of the first substrate 162 and/or a fold line of the second substrate 164 may define at least a portion of the waist opening 110.

It is to be appreciated that the first elastic belt 106 and the second elastic belt 108 may comprise the same materials and/or may have the same structure. In some embodiments, the first elastic belt 106 and the second elastic belt may comprise different materials and/or may have different structures. It should also be appreciated that components of the first elastic belt 106 and the second elastic belt 108, such as the first substrate 162, and/or second substrate 164 may be constructed from various materials. For example, the first and/or second belts may include a first substrate 162, and/or second substrate 164 that may be manufactured from materials such as plastic films; apertured plastic films; woven or nonwoven webs of natural materials (e.g., wood or cotton fibers), synthetic fibers (e.g., polyolefins, polyamides, polyester, polyethylene, or polypropylene fibers) or a combination of natural and/or synthetic fibers; or coated woven or nonwoven webs. In some configurations, the first and/or second belts may include a first substrate 162, and/or second substrate 164 comprising a nonwoven web of synthetic fibers, and may include a stretchable nonwoven. In some configurations, the first and second elastic belts may include an inner hydrophobic, non-stretchable nonwoven material and an outer hydrophobic, non-stretchable nonwoven material. It is to be appreciated that the belts may configured in various ways, such as disclosed for example, in U.S. Patent Application No. 63/111,790 and Chinese Patent Application No. CN2021/077843.

Elastic material 167 may be positioned between the wearer facing surface 162d of the first substrate 162 and the garment facing surface 164c of the second substrate 164. It is to be appreciated that the elastic material 167 may include one or more elastic elements such as strands, ribbons, elastic films, or panels extending along the lengths of the elastic belts. As shown in Figures 2A and 3, the elastic material 167 may include a plurality of elastic strands 168.

It is also to be appreciated that the first substrate 162, second substrate 164, and/or elastic material 167 of the first elastic belt 106 and/or second elastic belt 108 may be bonded together and/or with other components, such as the chassis 102, with adhesive and/or mechanical bonds. It is to be appreciated that adhesive and mechanical bonding methods may be utilized alone or in combination with each other.

In some configurations, adhesive may be applied to at least one of the first substrate 162, second substrate 164, and/or elastic material 167 when being combined to form the first elastic belt 106 and/or second elastic belt 108. In some configurations, mechanical bonding devices may apply mechanical bonds to the to at least one of the first substrate 162, second substrate 164, and/or elastic material 167 when being combined to form the first elastic belt 106 and/or second elastic belt 108. Such mechanical bonds may be applied with heat, pressure, and/or ultrasonic devices. In some configurations, mechanical bonding devices may apply bonds that bond the first substrate 162, second substrate 164, and/or elastic material 167 together and/or may act to trap or immobilize discrete lengths of the contracted elastic strands in the first elastic belt 106 and/or second elastic belt 108.

It is also to be appreciated that the first substrate 162, second substrate 164, and/or elastic material 167 may be bonded together with various methods and apparatuses to create various elastomeric laminates, such as described in U.S. Patent Publication Nos. 2018/0168878 A1; 2018/0168877 A1; 2018/0168880 A1; 2018/0170027 A1; 2018/0169964 A1; 2018/0168879 A1; 2018/0170026 A1; 2018/0168889 A1; 2018/0168874 A1; 2018/0168875 A1; 2018/0168890 A1; 2018/0168887 A1; 2018/0168892 A1; 2018/0168876 A1; 2018/0168891 A1; 2019/0070042 A1; and 2019/0070041 A1.

It is to be appreciated that components of the first elastic belt 106 and/or the second elastic belt 108 may be assembled in various ways and various combinations to create various desirable various features that may differ along the lateral width and/or longitudinal length of the first elastic belt 106 and/or the second elastic belt 108. Such features may include, for example, Dtex values, bond patterns, aperture arrangements, elastic positioning, Average Dtex values, Average Pre-Strain values, rugosity frequencies, rugosity wavelengths, height values, and/or contact area. It is to be appreciated that differing features may be imparted to various components, such as for example, the first substrate 162, second substrate 164, and elastic material 167 before and/or during stages of assembly of the first elastic belt 106 and/or the second elastic belt 108.

It is to be appreciated that the first elastic belt 106 and/or the second elastic belt 108 may include various configurations of belt elastic materials 167 arranged in relation to each other and to the first substrate 162, and the second substrate 164. As discussed above, the elastic material 167 may include configurations of one or more elastic elements such as strands, ribbons, films, or panels positioned in various arrangements. In some configurations, the elastic material 167 may comprise various elastics, elastic features and arrangements, and processes for assembly, such as described in 2018/0168889 A1; 2018/0168874 A1; 2018/0168875 A1; 2018/0168890 A1; 2018/0168887 A1; 2018/0168892 A1; 2018/0168876 A1; 2018/0168891 A1; 2019/0298586 A1; 2019/0070042 A1; 2018/0168878 A1; 2018/0168877 A1; 2018/0168880 A1; 2018/0170027 A1; 2018/0169964 A1; 2018/0168879 A1; 2018/0170026 A1; and 2019/0070041 A1. It is also to be appreciated the elastic materials 167 herein may be configured with identical or different colors in various different locations on the first elastic belt 106 and/or the second elastic belt 108.

In some configurations, the elastic material 167 may be configured as elastic strands 168 disposed at a constant interval in the longitudinal direction. In other embodiments, the elastic strands 168 may be disposed at different intervals in the longitudinal direction. In some configurations, the elastic material 167 in a stretched condition may be interposed and joined between uncontracted substrate layers. When the elastic material 167 is relaxed, the elastic material 167 returns to an unstretched condition and contracts the substrate layers. The elastic material 167 may provide a desired variation of contraction force in the area of the ring-like elastic belt. It is to be appreciated that the chassis 102 and elastic belts 106, 108 may be configured in different ways other than as depicted in attached Figures. It is also to be appreciated that the elastic material 167 material may be joined to the substrates continuously or intermittently along the interface between the elastic material 167 material and the substrates. In some configurations, the elastic strands 168 may be in the form of extruded elastic strands, which may also be bonded with the first substrate 162 and/or second substrate 164 in a pre-corrugated configuration, such as disclosed for example in U.S. Patent No. 5,681,302.

As discussed above for example with reference to Figures 2A and 3, the elastic material 167 discussed herein may be in the form of elastic strands 168. In some configurations, the elastic strands 168 may be parallel with each other and/or with the lateral axis 126. It is to be appreciated that the first elastic belt 106 and/or second elastic belt 108 may be configured to include various quantities of elastic strands 168. In some configurations, the first elastic belt 106 and/or second elastic belt 108 may comprise from about 100 to about 1500 elastic strands 168. It is also to be appreciated that elastic strands 168 herein may comprise various Dtex values, strand spacing values, and pre-strain values and such elastic strands 168 may utilized with other elastic strands to create first and second elastic belts 106, 108 comprising elastic strands 168 in various combinations of Dtex values, strand spacing values, and pre-strain values. For example, in some configurations, the Average-Dtex of one or more elastic strands 168 may be greater than 500. In some configurations, the Average-Dtex of one or more elastic strands 168 may be from about 10 to about 500, specifically reciting all 1 Dtex increments within the above-recited range and all ranges formed therein or thereby. In some configurations, a plurality of elastic strands 168 may comprise an Average-Strand-Spacing of less than or equal to 4 mm. In some configurations, a plurality of elastic strands 168 may comprise an Average-Strand-Spacing from about 0.25 mm to about 4 mm, specifically reciting all 0.01 mm increments within the above-recited range and all ranges formed therein or thereby. In some configurations, a plurality of elastic strands 168 may comprise an Average-Strand-Spacing of greater than 4 mm. In some configurations, the Average-Pre-Strain of each of a plurality of elastic strands may be from about 50% to about 400%, specifically reciting all 1% increments within the above-recited range and all ranges formed therein or thereby. In some configurations, the elastic strands 168 comprise an Average-Strand-Spacing from about 0.25 mm to about 4 mm and an Average-Dtex from about 10 to about 500. In some configurations, the elastic strands 168 may comprise an Average-Pre-Strain from about 75% to about 300%.

In some configurations, a first plurality of elastic strands may comprise a first Average-Pre-Strain from about 75% to about 300%, and a second plurality of elastic strands may comprise a second Average-Pre-Strain that is greater than first Average-Pre-Strain. In some configurations, a first plurality of elastic strands comprises an Average-Strand-Spacing from about 0.25 mm to about 4 mm and an Average-Dtex from about 10 to about 500; and a second plurality of elastic strands may comprise an Average-Strand-Spacing greater than about 4 mm and an Average-Dtex greater than about 450.

In some configurations, such as shown in Figure 2A, the elastic strands 168 may be referred to herein as outer, waist elastics 170 and inner, waist elastics 172. Elastic strands 168, such as the outer waist elastics 170, may continuously extend laterally between the first and second opposing end regions 106a, 106b of the first elastic belt 106 and between the first and second opposing end regions 108a, 108b of the second elastic belt 108. Some elastic strands 168, such as the inner waist elastics 172, may be configured with discontinuities in areas, such as for example, where the first and second elastic belts 106, 108 overlap portions of the chassis 102, such as the absorbent assembly 140.

As shown in Figure 2A, the first elastic belt 106 and/or the second elastic belt 108 may be configured with high-stretch zones 400 and low-stretch zones 402. The first elastic belt 106 and/or the second elastic belt 108 may include a first high-stretch zone 400a and a second high-stretch zone 400b separated laterally by a low-stretch zone 402. Portions of the chassis 102, such as the absorbent assembly 140, may be connected with the first elastic belt 106 and/or the second elastic belt 108 in the low-stretch zones 402 in the first waist region 116 and/or the second waist region 118. The high-stretch zones 400 are elasticated by the elastic material 167, such as the elastic strands 168, 172; and the low-stretch zones 402 comprise cut lines 404 separating the elastic material 167, such as the elastic strands 168, 172, into discrete pieces 406. In turn, the low-stretch zones 402 define regions of the first elastic belt 106 and/or the second elastic belt 108 that have relatively less elasticity than the high-stretch zones 400. The discrete elastic pieces 406 that are separated from each other and which are elastically contracted do not add any substantial amount of elastication to the low-stretch zone 402. As such, upon application of a force, the high-stretch zones 400 will elongate more than the low-stretch zones 402. As provided above, the terms "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force. In some configurations, the first elastic belt 106 and/or the second elastic belt 108 may be configured with high-stretch zones 400 that are elastic and may be configured with low-stretch zones 402 that are not elastic or "inelastic."

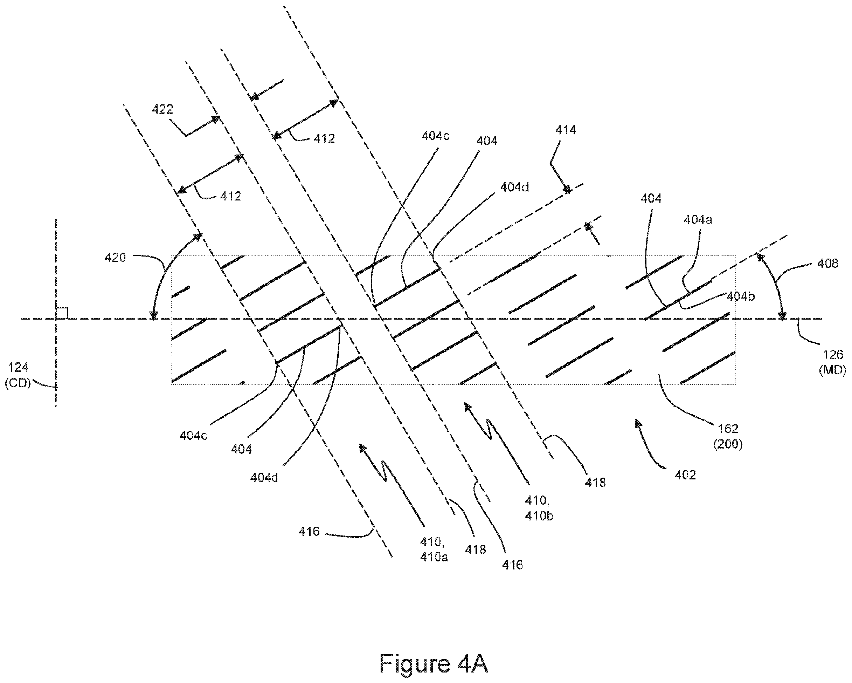

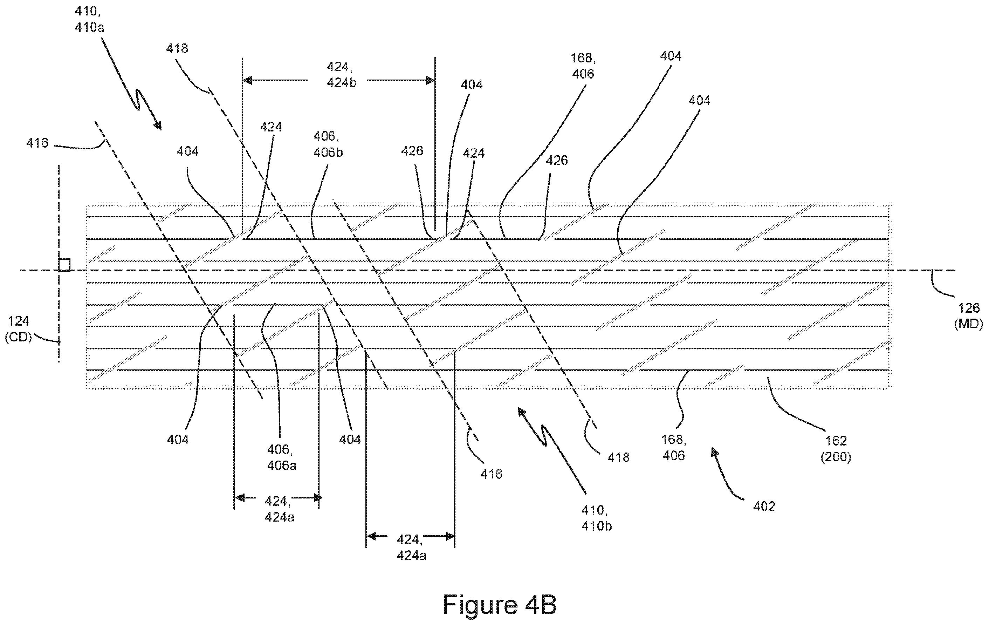

Figures 4A and 4B show detailed views of portions of low-stretch zones 402 such as shown, for example, in the first belt 106 and the second belt of the pant diaper 100p illustrated in Figure 2A. As shown in Figures 4A and 4B, cut lines 404 in the low-stretch zones 402 separate the elastic strands 168 into discrete pieces 406. For the purposes of clarity, the discrete pieces 406 of elastic strands 168 are not shown in Figure 4A, and the cut lines 404 are faded in Figure 4B to accentuate the visibility of the discrete pieces 406 of elastic strands 168. In some configurations, the cut lines 404 penetrate through the elastic strands 168, the first substrate 162, and the second substrate 164. In some configurations, the cut lines 404 may penetrate through the elastic strands 168 and only one of the first substrate 162 and the second substrate 164. It is to be appreciated that the cut lines 404 may be arranged in various orientations and sizes. For example, as shown in Figure 4A the cut lines may be oriented to define an offset angle 408 relative to the lateral axis 126. The size of the offset angle 408 may be configured to help minimize or prevent the separation of opposing sides 404a, 404b of the cut lines 404 when the low-stretch zone 402 is subjected to opposing forces in the lateral directions, such as when the elastic belts 106, 108 are stretched laterally. In some configurations, offset angles 408 may be greater than 0 degrees and less than 45 degrees, specifically reciting all 1 degree increments within the above-recited range and all ranges formed therein or thereby.

With continued reference to Figure 4A, the cut lines 404 may be arranged in rows 410 comprising at least a first row 410a and a second row 410b neighboring the first row 410a. The cut lines in the first row 410a and the second row 410b may extend for a length 412 from a first end 404c to a second end 404d. In some configurations, the length 412 of each cut line 404 in the first row 410a and the second row 410b may be about 4 mm. In some configurations, the cut lines 404 in the first row 410a and the cut lines 404 in the second row 410b are parallel to each other. The 404 cut lines in the first row 410a and/or second row 410b may be separated from each other by a cut line gap distance 414. In some configurations, the cut line gap distance 414 may be about 1.9 mm. The first ends 404c of cut lines 404 in the first row 410b and the first ends 404c of cut lines 404 in the second row 410b may be aligned along first reference lines 416 that are oriented to define a row angle relative 420 to the lateral axis 126. In some configurations, the row angle 420 may be less than 90 degrees and greater than 45 degrees, specifically reciting all 1 degree increments within the above-recited range and all ranges formed therein or thereby. As shown in Figure 4A, the second ends 404d of cut lines 404 in the first row 410a and the second ends 404d of cut lines 404 in the second row 410b may be aligned along second reference lines 418. In some configurations, the second reference 418 lines are parallel to the first reference lines 416. In addition, the second reference line 418 of the first row 410a may be separated from the first reference line 416 of the second row 410b by a row gap distance 422. In some configurations, the row gap distance 422 may be about 1.9 mm.

As previously mentioned, the cut lines 404 in the low-stretch zones 402 separate the elastic strands 168 into discrete pieces 406. As shown in Figure 4B, the discrete pieces 406 of elastic strands 168 extend for an elastic piece length 422 between a first end 424 and a second end 426. Discrete pieces 406 may extend laterally such that first ends 424 and second ends 426 of laterally neighboring pieces 406 are separated from each other by cut lines 404. Because the discrete pieces 406 are cut from the elastic strands 168, it is also be appreciated that the discrete pieces 406 may be spaced from each other in the longitudinal direction with the same spacing as the elastic strands 168 and may be arranged in a parallel relationship with each other. Figure 4B also shows a detailed view of elastic strands 168 that are separated into first discrete pieces 406a and second discrete pieces 406b by the cut lines 404. The first discrete pieces 406a may comprise a first elastic piece length 422a and the second discrete pieces 406b may comprise a second elastic piece length 422b. In some configurations, the second elastic piece length 422b is greater than the first elastic piece length 422a. In some configurations, the first elastic piece length 422a of the first discrete pieces 406a may be defined by a distance extending laterally between neighboring cut lines in the first row 410a, and the second elastic piece length 422b of the second discrete pieces 406b may be defined by a distance extending laterally between cut lines 404 in the first row 410a and cut lines 404 in the second row 410b. In some configurations, a ratio of the second elastic piece length 422b to the first elastic piece length 422a is about 2: 1. As previously mentioned, the elastic strands 168 may be continuously bonded with at least one of the first substrate 162 and the second substrate 164 or may be intermittently bonded with at least one of the first substrate 162 and the second substrate 164. As such, the discrete pieces 406 of elastic strands 168 may be continuously bonded with at least one of the first substrate 162 and the second substrate 164 or may be intermittently bonded with at least one of the first substrate 162 and the second substrate 164. It is also to be appreciated that the elastic strands 168 and the discrete pieces 406 of elastic strands 168 may be bonded with adhesive applied to at least one of the first substrate 162, the second substrate 164, and the elastic strands 168.

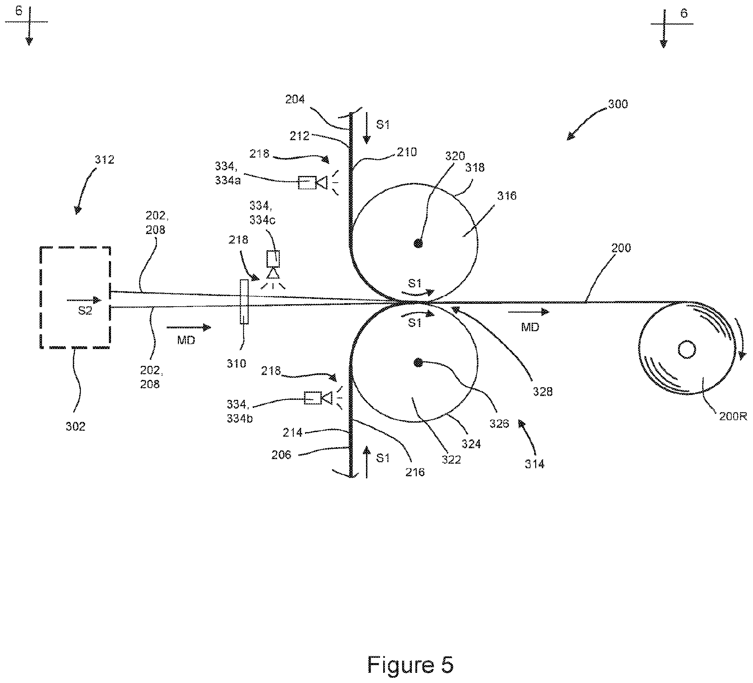

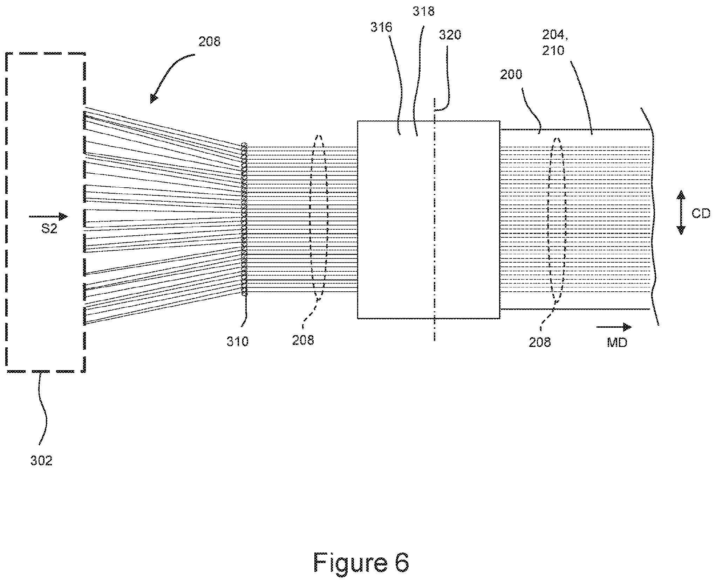

As previously mentioned, various apparatuses and methods may be utilized to produce elastomeric laminates according to the present disclosure that may be used to construct various components of diapers, such as elastic belts, leg cuffs, and the like. For example, Figures 5 and 6 show schematic views of a converting apparatus 300 adapted to manufacture elastomeric laminates 200. As described in more detail below, the converting apparatus 300 shown in Figures 5 and 6 operates to advance a continuous length of elastic material 202, a continuous length of a first substrate 204, and a continuous length of a second substrate 206 along a machine direction MD. It is also to be appreciated that in some configurations, the first substrate and second substrate 204, 206 herein may be defined by two discrete substrates or may be defined by folded portions of a single substrate. The apparatus 300 stretches the elastic material 202 and joins the stretched elastic material 202 with the first and second substrates 204, 206 to produce an elastomeric laminate 200. Although the elastic material 202 is illustrated and referred to herein as strands 208, it is to be appreciated that in some configurations, elastic material 202 may include one or more continuous lengths of elastic strands, ribbons, and/or films.

It is to be appreciated that the elastomeric laminates 200 can be used to construct various types of absorbent article components. It also to be appreciated that the methods and apparatuses herein may be adapted to operate with various types of absorbent article assembly processes, such as disclosed for example in U.S. Patent Publication Nos. 2013/0255861 A1; 2013/0255862 A1; 2013/0255863 A1; 2013/0255864 A1; and 2013/0255865 A1. For example, the elastomeric laminates 200 may be used as a continuous length of elastomeric belt material that may be converted into the first and second elastic belts 106, 108 discussed above with reference to Figures 1A-4B. As such, the elastic material 202 may correspond with the belt elastic material 168 interposed between the outer layer 162 and the inner layer 164, which in turn, may correspond with either the first and/or second substrates 204, 206. In other examples, the elastomeric laminates 200 may be used to construct waistbands and/or side panels in taped diaper configurations. In yet other examples, the elastomeric laminates 200 may be used to construct various types of leg cuff and/or topsheet configurations.

It is to be appreciated that elastic laminate 200 may be assembled and/or supplied in various ways. For example, Figures 5 and 6 show an example of a converting apparatus 300 for producing an elastomeric laminate 200 that may include a first metering device 312 and a second metering device 314. The first metering device 312 may be configured as an elastic strand supply apparatus, such as one or more unwinders 302 generically represented by a dash line rectangle, that may include one or more spools of elastic strands 208. During operation, the elastic strands 208 advance in the machine direction MD from the unwinder 302 to the second metering device 314. In addition, the elastic strands 208 may be stretched along the machine direction MD while advancing between the unwinder 302 and the second metering device 314. The stretched elastic strands 208 are also joined with the first substrate 204 and the second substrate 206 at the second metering device 314 to produce an elastomeric laminate 200. It is to be appreciated that the elastic strands 208 may advance along and/or around one or more guide rollers. It is also to be appreciated that the elastic strands 208 may be stretched along a continuous path while advancing in the machine direction MD or may be stretched in various steps that provide multiple increases in elongation while advancing in the machine direction MD.

As shown in Figure 5, the second metering device 314 may include: a first roller 316 having an outer circumferential surface 318 and rotates about a first axis of rotation 320, and a second roller 322 having an outer circumferential surface 324 and rotates about a second axis of rotation 326. The first roller 316 and the second roller 322 rotate in opposite directions, and the first roller 316 is adjacent the second roller 322 to define a nip 328 between the first roller 316 and the second roller 322. The first roller 316 may rotate such that the outer circumferential surface 318 has a surface speed S1, and the second roller 322 may rotate such that the outer circumferential surface 324 has the same, or substantially the same, surface speed S1.

As shown in Figure 5, the first substrate 204 includes a first surface 210 and an opposing second surface 212, and the first substrate 204 advances to the first roller 316. In particular, the first substrate 204 advances at speed S1 to the first roller 316 where the first substrate 204 partially wraps around the outer circumferential surface 318 of the first roller 316 and advances through the nip 328. As such, the first surface 210 of the first substrate 204 travels in the same direction as and in contact with the outer circumferential surface 318 of the first roller 316. In addition, the second substrate 206 includes a first surface 214 and an opposing second surface 216, and the second substrate 206 advances to the second roller 322. In particular, the second substrate 206 advances at speed S1 to the second roller 322 where the second substrate 206 partially wraps around the outer circumferential surface 324 of the second roller 322 and advances through the nip 328. As such, the second surface 216 of the second substrate 206 travels in the same direction as and in contact with the outer circumferential surface 324 of the second roller 322. It is to be appreciated that the first and/or substrates 204, 206 may advance at various speeds S1. In some configurations, the first substrate 204 and/or the second substrate 206 may advance at speed S1 from about 150 meters/minute to about 500 meters/minute, specifically reciting all 1 meter/minute increments within the above-recited range and all ranges formed therein or thereby.

In some configurations, the elastic strands 208 may also be stretched in the machine direction MD and combined with the first substrate 204 and the second substrate 206 in the stretched state. For example, with continued reference to Figures 5 and 6, the unwinder 302 may unwind or otherwise supply the elastic strands 208 advancing at a speed S2 in the machine direction MD to the nip 328. In some configurations, the speed S2 is less than the speed S1, and as such, the elastic strands 208 are stretched in the machine direction MD. In turn, the stretched elastic strands 208 advance through the nip 328 between the first and second substrates 204, 206 such that the elastic strands 208 are joined with the second surface 212 of the first substrate 204 and the first surface 214 of the second substrate 206 to produce a continuous length of elastomeric laminate 200.

As previously mentioned, the apparatus 300 may include an elastic strand supply apparatus, such as one or more unwinders 302, that supplies a plurality of elastic strands 208. It is to be appreciated the unwinders 302 herein may be configured in various ways. For example, the unwinder 302 may be configured with individual spools with mandrel and/or surface driven unwinders, overend unwinders, and/or beam unwinders (also referred to as warp beams). Various types of unwinders are disclosed in U.S. Patent Nos. 6,676,054; 7,878,447; 7,905,446; 9,156,648; 4,525,905; 5,060,881; and 5,775,380; U.S. Patent Application No. 17/189,476, filed on March 2, 2021; and U.S. Patent Publication Nos. 2004/0219854 A1; 2018/0168879 A1; and 2018/0170026 A1. Additional examples of elastics and associated handling equipment are available from Karl Mayer Corporation. It is to be appreciated that the apparatus 300 may be configured to assemble elastomeric laminates 200 with elastic strands 208 unwound from more than one unwinder 302 in combination with elastic strands supplied from the same and/or different types of elastic unwinder configurations. It is also to be appreciated that the elastic strands 208 may include various types of spin finish, also referred herein as yarn finish, configured as coating on the elastic strands 208 that may be intended to help prevent the elastic strands from adhering to themselves, each other, and/or downstream handling equipment. As such, the apparatus may also be configured to remove or partially remove the spin finish from the elastic strands, such as disclosed for example in U.S. Patent Publication No. 2018/0168877 A1.

As previously mentioned, the apparatus 300 may include one or more unwinders 302 that may supply various quantities of elastic strands. In some configurations, the unwinders 302 herein may include from 1 to about 3000 spools positioned thereon, and thus, may have from 1 to about 3000 elastic strands 208 advancing therefrom, specifically reciting all 1 spool and strand increments within the above-recited range and all ranges formed therein or thereby. In turn, the elastomeric laminates 200 herein may include from 1 to about 3000 elastic strands 208 spaced apart from each other in the cross direction CD, specifically reciting all 1 elastic strand increments within the above-recited range and all ranges formed therein or thereby.

It is also to be appreciated that the apparatuses and processes may be configured such that elastic strands 208 may be advanced from the unwinders 302 and directly to the assembly process without having to touch additional machine components, such as for example, guide rollers. It is also to be appreciated that in some configurations, elastic strands 208 may be advanced from the unwinders 302 and may be redirected and/or otherwise touched by and/or redirected by machine components, such as for example guide rollers, before advancing to the assembly process.

As shown in Figures 5 and 6, the elastic strands 208 may also advance through a strand guide 310 before being combined with the first substrate 204 and the second substrate 206. The strand guide 310 may space or separate neighboring elastic strands 208 from each other at a desired distance in a cross direction CD before being combined with the first substrate 204 and the second substrate 206. As shown in Figures 5 and 6, the elastic strands may advance through a strand guide 310 positioned between the unwinder 302 and the nip 328. The strand guide 310 may operate to change and/or dictate and/or fix the cross directional CD separation distance between neighboring elastic strands 208 advancing into the nip 328 and in the assembled elastomeric laminate 200. It is to be appreciated that the elastic strands 208 may be separated from each other by various distances in the cross direction CD advancing into the nip 328 and in the assembled elastomeric laminate 200. In some configurations, neighboring elastic strands 208 may be separated from each other by about 0.5 mm to about 4 mm in the cross direction CD, specifically reciting all 0.1 mm increments within the above-recited range and all ranges formed therein or thereby. It is to be appreciated that the strand guide 310 may be configured in various ways. In some configurations, the strand guide 310 may be configured as a comb that may comprise a plurality of tines or reeds. In turn, the advancing elastic strands 208 are separated and spaced apart from each other by the tines or reeds in the cross direction CD from each other. In some configurations, the strand guide 310 may include a plurality of rollers that separate and space the elastic strands in the cross direction CD from each other.

It is to be appreciated that different components may be used to construct the elastomeric laminates 200 in accordance with the methods and apparatuses herein. For example, the first and/or second substrates 204, 206 may include nonwovens and/or films. In addition, the elastic strands 208 may be configured in various ways and may have various decitex values. In some configurations, the elastic strands 208 may be configured with decitex values ranging from about 10 decitex to about 500 decitex, specifically reciting all 1 decitex increments within the above-recited range and all ranges formed therein or thereby.

As discussed above, it is to be appreciated that the elastomeric laminates 200 assembled herein may include various quantities of elastic strands 208 spaced apart from each other by various distances and may include various decitex values. For example, the elastomeric laminates 200 herein may have various elastic densities, wherein the elastic density may be defined as decitex per elastomeric laminate width. For example, some elastomeric laminates 200 may have an elastic density from about 30 decitex/mm to about 150 decitex/mm, specifically reciting all 1 decitex/mm increments within the above-recited range and all ranges formed therein or thereby. In another example, the elastomeric laminates 200 herein may have various numbers of elastic strands arranged in the cross direction CD per meter of elastomeric laminate cross directional width. For example, some elastomeric laminates 200 may have from about 500 elastic strands/meter of elastomeric laminate width to about 2000 elastic strands/meter of elastomeric laminate width, specifically reciting all 1 elastic strand/meter increments within the above-recited range and all ranges formed therein or thereby.

As shown in Figure 5, the apparatus 300 may include one or more adhesive applicator devices 334 that may apply adhesive 218 to at least one of the elastic strands 208, the first substrate 204, and the second substrate 206 before being combined to form the elastomeric laminate 200. For example, the first substrate 204 may advance past an adhesive applicator device 334a that applies adhesive 218 to the second surface 212 of the first substrate 204 before advancing to the nip 328. It is to be appreciated that the adhesive 218 may be applied to the first substrate 204 upstream of the first roller 316 and/or while the first substrate 204 is partially wrapped around the outer circumferential surface 318 of the first roller 316. In another example, the second substrate 206 may advance past an adhesive applicator device 334b that applies adhesive 218 to the first surface 214 of the second substrate 206 before advancing to the nip 328. It is to be appreciated that the adhesive 218 may be applied to the second substrate 206 upstream of the second roller 322 and/or while the second substrate 206 is partially wrapped around the outer circumferential surface 324 of the second roller 324. In another example, an adhesive applicator device 334c may be configured to apply adhesive 218 to the elastic strands 208 before and/or while being joined with first substrate 204 and second substrate 206.

It is to be appreciated that the adhesive applicator devices herein 334 be configured in various ways, such as for example, spray nozzles and/or slot coating devices. In some configurations, the adhesive applicator devices 334 may be configured in accordance with the apparatuses and/or methods disclosed in U.S. Patent Nos. 8,186,296; 9,265,672; 9,248,054; and 9,295,590 and U.S. Patent Publication No. 2014/0148773 A1.

It is to be appreciated that the elastic strands 208 may be joined to the first substrate 204 and/or the second substrate 206 continuously or intermittently along the interface between the elastic strands 208 material and the substrates 204, 206. In some configurations, adhesive 334 may be applied intermittently on the first substrate 204 and/or the second substrate 206 to create intermittent bonds along the lengths of the elastic strands 208 between the first substrate 204 and/or the second substrate 206. It is to be appreciated that intermittent application of adhesive 334 may be created with spray nozzles and/or slot coat devices that apply intermittent patterns of adhesive 334. In some configurations, adhesive 334 may be applied intermittently along the length of the advancing elastic strands 208 to create intermittent bonds along the lengths of the elastic strands 208 between the first substrate 204 and/or the second substrate 206. In some configurations, slot coat devices may be configured to continuously apply adhesive 334 at relatively low basis weights onto the first substrate 204 and/or the second substrate 206, wherein the relatively low basis weights of adhesive results in the creation of intermittent bonding between the first substrate 204 and/or the second substrate 206 along the lengths of the elastic strands 208. In some configurations, slot coat devices may be configured to continuously apply adhesive 334 at relatively high basis weights onto the first substrate 204 and/or the second substrate 206, wherein the relatively high basis weights of adhesive results in the creation of continuous bonding between the first substrate 204 and/or the second substrate 206 along the lengths of the elastic strands 208. In some configurations, adhesive 334 may be applied continuously along the length of the advancing elastic strands 208 to create continuous bonds along the lengths of the elastic strands 208 between the first substrate 204 and/or the second substrate 206.

In some configurations, the apparatus 300 may include a mechanical bonding device that applies the mechanical bonds to the elastomeric laminate 200, such as for example, bonds that may be applied with heat, pressure, and/or ultrasonic devices. Examples of ultrasonic bonding devices, which may include linear or rotary type configurations, are disclosed for example in U.S. Patent Nos. 3,113,225; 3,562,041; 3,733,238; 5,110,403; 6,036,796; 6,508,641; and 6,645,330. In some configurations, the ultrasonic bonding device may be configured as a linear oscillating type sonotrode, such as for example, available from Herrmann Ultrasonic, Inc. Additional examples of mechanical bonding devices and methods are disclosed in U.S. Patent Nos. 4,854,984; 6,291,039; 6,248,195; 8,778,127; and 9,005,392; and U.S. Patent Publication Nos. 2014/0377513 A1; and 2014/0377506 A1. It is to be appreciated that the mechanical bonding device may apply mechanical bonds to the elastomeric laminate at or downstream of the nip 328. The mechanical bonding device may apply bonds that bond the first substrate 204, the second substrate 206, and/or elastic strands 208 together and/or may act to trap or immobilize discrete lengths of the contracted elastic strands 208 in the elastomeric laminate 200. It is also to be appreciated that the apparatuses herein may include one of, some of, or all of adhesive applicator devices 334a, 334b, 334c and mechanical bonding device mentioned herein.

It is also to be appreciated that the elastic strands 208 may be bonded with the first substrate 204 and/or second substrate 206 with various methods and apparatuses to create various elastomeric laminates, such as described in U.S. Patent Publication Nos. 2018/0168878 A1; 2018/0168877 A1; 2018/0168880 A1; 2018/0170027 A1; 2018/0169964 A1; 2018/0168879 A1; 2018/0170026 A1; 2018/0168889 A1; 2018/0168874 A1; 2018/0168875 A1; 2018/0168890 A1;

2018/0168887 A1; 2018/0168892 A1; 2018/0168876 A1; 2018/0168891 A1; 2019/0070042 A1; and 2019/0070041 A1.

It is to be appreciated that the apparatuses 300 herein may be configured in various ways with various features described herein to assemble elastomeric laminates 200 having various stretch characteristics. For example, when the elastomeric laminate 200 is elongated, some elastic strands 208 may exert contraction forces in the machine direction MD that are different from contraction forces exerted by other elastic strands 208. Such differential stretch characteristics can be achieved by stretching some elastic strands 208 more or less than other elastic strands 208 before joining the elastic strands with the first and second substrates 204, 206. As discussed above, the spools of elastic strands 208 may be unwound from one or more unwinders 302 at different speeds from each other, and as such, the elastic strands 208 may be stretched more or less than each when combined with the first and second substrates. For example, as previously discussed, the first substrate 204 and the second substrate 206 may each advance at a speed S1. In some configurations, the some elastic strands 208 may advance at speed S2 that is less than the speed S1 are also different from the advancement speeds of other elastic strands. As such, some elastic strands 208 are stretched by different amounts in the machine direction MD when combined with the first and second substrates 204, 206.

As discussed herein, the elastic strands 208 may be pre-strained prior to joining the elastic strands 208 to the first or second substrate layers 204, 206. In some configurations, the elastic strands 208 may be pre-strained from about 75% to about 300%, specifically reciting all 1% increments within the above-recited range and all ranges formed therein or thereby. In some configurations, the elastic strands 208 may be pre-strained from about 80% to about 250%, specifically reciting all 1% increments within the above-recited range and all ranges formed therein or thereby. Pre-strain refers to the strain imposed on an elastic or elastomeric material prior to combining it with another element of the elastomeric laminate or the absorbent article. Pre-strain is determined by the following equation: Pre-strain = ((extended length of the elastic-relaxed length of the elastic)/relaxed length of the elastic)*100.

It is also to be appreciated that the elastic strands 208 may have various different material constructions and/or decitex values to create elastomeric laminates 200 having different stretch characteristics in different regions. In some configurations, the spools of elastic strands 208 having different decitex values may be positioned on and advanced from one or more unwinders 302. In some configurations, the elastomeric laminate 200 may have regions where the elastic strands 208 are spaced relatively close to one another in the cross direction CD and other regions where the elastic strands 208 are spaced relatively far apart from each other in the cross direction CD to create different stretch characteristics in different regions. In some configurations, the elastic strands 208 may be supplied on the spool in a stretched state, and as such, may not require additional stretching (or may require relatively less additional stretching) before being combined with the first substrate 204 and/or the second substrate 206. In some configurations, differential stretch characteristics in an elastomeric laminate 200 may be created by bonding another substrate and/or elastomeric laminate and/or an elastic film to a particular region of an elastomeric laminate. In some configurations, differential stretch characteristics in an elastomeric laminate 200 may be created by folding a portion of an elastomeric laminate onto itself in a particular region of the elastomeric laminate.

In some configurations, the elastic strands 208 may be joined with the first and second substrates 204, 206 such that the elastomeric laminate 200 may have different stretch characteristics in different regions along the cross direction CD, such as disclosed in U.S. Patent Publication Nos. 2006/0094319A1; 2006/0032578A1; 2018/0168878 A1; 2018/0168877 A1; 2018/0168880 A1; 2018/0170027 A1; 2018/0169964 A1; 2018/0168879 A1; 20180170026 A1; 2018/0168889 A1; 2018/0168874 A1; 2018/0168875 A1; 2018/0168890 A1; 2018/0168887 A1; 2018/0168892 A1; 2018/0168876 A1; 2018/0168891 A1; 2019/0070042 A1; and 2019/0070041 A1,. In some configurations, the elastomeric laminate 200 may include different tension zones that may help make some web handling operations less cumbersome, such as disclosed in U.S. Patent Publication No. 2002/0009940 A1.

As shown in Figure 6, the elastomeric laminate 200 may advance from the nip 328 and may be accumulated, such as for example, by being wound onto a roll 200R or being festooned in a container. It is to be appreciated that the elastomeric laminate 200 may be wound onto a roll 200R in a fully stretched, partially stretched, or fully relaxed state. The accumulated elastomeric laminate 200 may be stored and/or moved to a location for incorporation into an absorbent article assembly process, wherein the elastomeric laminate 200 may be converted into an absorbent article component, such as discussed above. As such, the accumulated elastomeric laminate 200 may be unwound from a roll 200R (or drawn from a container) and incorporated into an absorbent article assembly line. It is to be appreciated that the apparatus 300 may be configured to assemble elastomeric laminates 200 that may be cut along the machine direction MD to define separate lanes of elastic of individual elastomeric laminates 200. In some configurations, the elastomeric laminate may be cut into separate lanes of individual elastomeric laminates 200 before wound onto respective rolls 200R. In some configurations, the elastomeric laminate may be cut into separate lanes of individual elastomeric laminates 200 as the elastomeric laminate is unwound from a roll 200R.

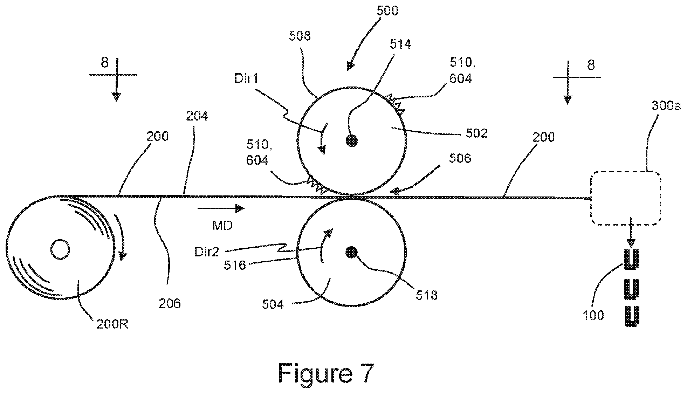

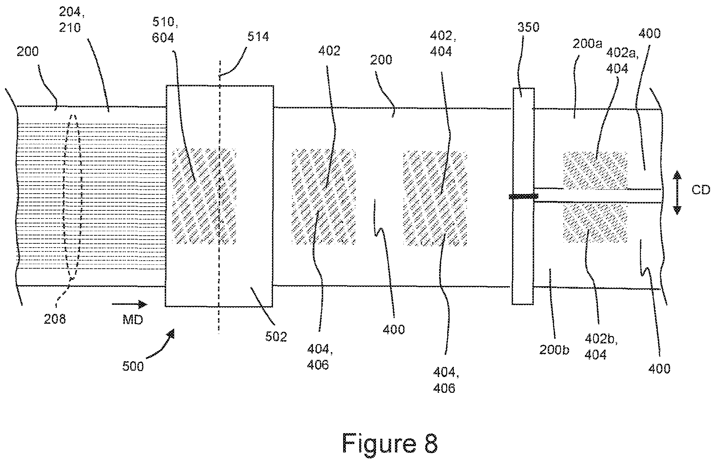

As shown in Figures 7 and 8, the elastic laminate 200 may be advanced in a machine direction through a cutting device 500 adapted to cut elastic strands 208 into discrete pieces 406 so as to create low-stretch zones 402 in the elastic laminate 200. Although the elastic laminate is depicted in Figure 7 as advancing from a roll 200R to the cutting device 500, it is to be appreciated that the elastic laminate 200 could be configured to advance directly from an assembly apparatus 300 such as described with reference to Figures 5 and 6 without first being accumulated.

It is also to be appreciated that in some configurations, the elastomeric laminate 200 may advance from the cutting device 500 and may then be accumulated as discussed above. In some configurations, the elastomeric laminate 200 may advance from the cutting device 500 and may be incorporated directly into an absorbent article assembly process. For example, Figure 7 shows the elastomeric laminate 200 advancing from the cutting device 500 directly into an absorbent article assembly line 300a, generically represented by rectangle in dashed lines. The absorbent article assembly may be configured to convert the elastic laminate 200 along with additional components to assemble absorbent articles 100, such as diapers discussed above with reference to Figures 1-4B for example.

With continued reference to Figures 7 and 8, the cutting device 500 may include a knife roll 502 positioned adjacent an anvil roll 504 to define a nip 506 therebetween. The knife roll 502 may include an outer circumferential surface 508 and blades 510 extending radially outward to blade edges 604 and adapted to rotate about an axis 514 in a first direction Dir1. The anvil roll 504 may include an outer circumferential surface 516 adapted to rotate about an axis 518 in a second direction Dir2 opposite the first direction Dir1. As the elastic laminate 200 advances through the nip 506 between the knife roll 502 and the anvil roll 504, the blades 510 operate to cut the elastic strands 208 into discrete pieces 406 separated from each other along the machine direction MD by cut lines 404 in low-stretch zones 402. In addition to cutting the elastic strands 208, the blades 510 may also cut through one or both the first substrate 204 and the second substrate 206. The low-stretch zones 402 may be separated from each other by high-stretch zones 400 along the machine direction MD, and wherein the high-stretch zones 400 may be separated from each other by low-stretch zones 402 along the machine direction MD. As opposed to or in addition to blades, it is to be appreciated that the cutting device 500 may be configured to perform cutting operations in various ways, such as lasers or ultrasonics for example.

It is to be appreciated that the high-stretch zones 400 and low-stretch zones 402 shown in the elastic laminate 200 shown in Figure 8 may correspond with the high-stretch zones 400 and low-stretch zones 402 in the first and/or second belts 106, 108 described above with reference to Figure 2A. As such, the high-stretch zones 400 are elasticated by the elastic material 202, such as the elastic strands 208; and the low-stretch zones 402 comprise cut lines 404 separating the elastic material 202, such as the elastic strands 208, into discrete pieces 406. In turn, the low-stretch zones 402 define regions of the elastic laminate 200 that have relatively less elasticity than the high-stretch zones 400. The discrete elastic pieces 406 that are separated from each other and which are elastically contracted do not add any substantial amount of elastication to the low-stretch zone 402. As such, upon application of a force, the high-stretch zones 400 will elongate more than the low-stretch zones 402. In some configurations, the elastic laminate 200 may be configured with high-stretch zones 400 that are elastic and may be configured with low-stretch zones 402 that are not elastic or "inelastic."

It is to be appreciated that the descriptions provided above with respect to details relating to the low-stretch zones 400 described with reference to Figures 4A and 4B are also applicable to the low-stretch zones 400 shown in Figure 8. When applying the descriptions of the low-stretch zones of the elastic belts 106, 108 in Figures 4A and 4B above to the low-stretch zones 402 of the elastic laminate 200 in Figure 7, the orientations of the cut lines 404, rows 410, and discrete pieces 406 relative to the lateral axis 126 and longitudinal axis 124 may be taken relative to the cross direction CD and the machine direction MD, respectively, of the elastic laminate 200.

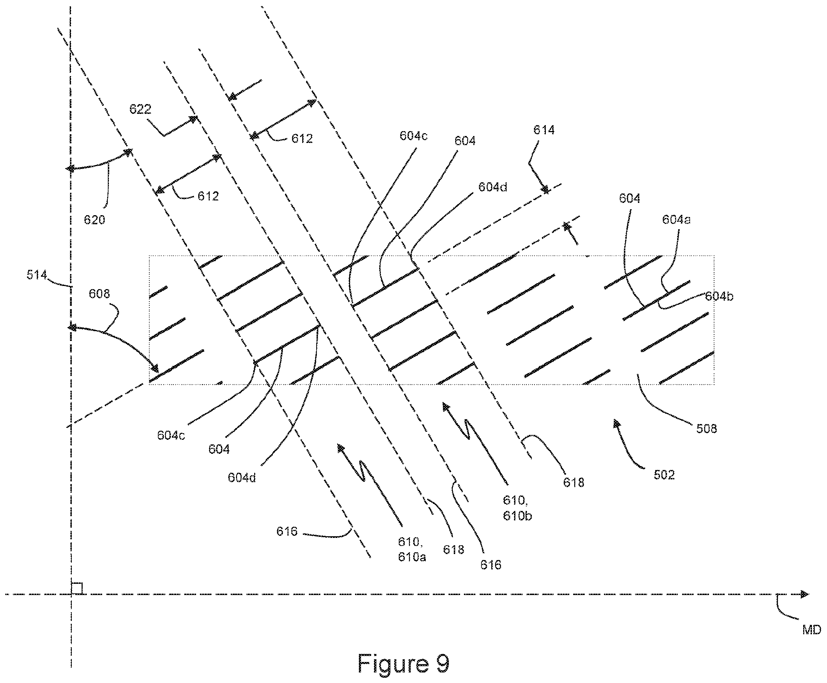

As mentioned above, the knife roll 502 may include blades 510 extending radially outward to blade edges 604 adapted to rotate about the axis 514. As such, the blade edges 604 may be oriented to cut the elastic strands 208 and one or both of the first substrate 204 and the second substrate 206 to create cut lines 404 in the elastic laminate 200 that correspond with cut lines 404 and discrete elastic pieces 406 described above with reference to Figures 4A and 4B. Figure 9 shows an example orientation of a portion of a group of blade edges 604 on a knife roll 502. It is to be appreciated that the knife roll 502 may be configured to rotate at a variable angular velocity or a constant angular velocity and may be driven by a servo motor.

It is to be appreciated that the blade edges 604 may be arranged in various orientations and sizes. For example, as shown in Figure 9 the blade edges 604 may be oriented to define an offset angle 608 relative to the rotation axis 514. In some configurations, offset angles 608 may be greater than 45 degrees and less than 90 degrees, specifically reciting all 1 degree increments within the above-recited range and all ranges formed therein or thereby. With continued reference to Figure 9, the blade edges 604 may be arranged in rows 610 comprising at least a first row 610a and a second row 610b neighboring the first row 610a. The blade edges in the first row 610a and the second row 610b may extend for a length 612 from a first end 604c to a second end 604d. In some configurations, the length 612 of each blade edge 604 in the first row 610a and the second row 610b may be about 4 mm. In some configurations, the blade edges 604 in the first row 610a and the blade edges 604 in the second row 610b are parallel to each other. The 604 blade edges in the first row 610a and/or second row 610b may be separated from each other by a blade edge gap distance 614. In some configurations, the blade edge gap distance 614 may be about 1.9 mm. The first ends 604c of blade edges 604 in the first row 610b and the first ends 604c of blade edges 604 in the second row 610b may be aligned along first reference lines 616 that are oriented to define a row angle relative 620 to the rotation axis 514. In some configurations, the row angle 620 may be less than 45 degrees and greater than 0 degrees, specifically reciting all 1 degree increments within the above-recited range and all ranges formed therein or thereby. As shown in Figure 9, the second ends 604d of blade edges 604 in the first row 610a and the second ends 604d of blade edges 604 in the second row 610b may be aligned along second reference lines 618. In some configurations, the second reference 618 lines are parallel to the first reference lines 616. In addition, the second reference line 618 of the first row 610a may be separated from the first reference line 616 of the second row 610b by a row gap distance 622. In some configurations, the row gap distance 622 may be about 1.9 mm.

In some configurations, the first elastic piece length 422a discussed above may be defined by a distance between blade edges 604 within the same row 610 or a distance between blade edges 604 in different rows 610. In some configurations, the second elastic piece length 422b may be defined only by a distance between blade edges 604 in different rows 610 as opposed to a distance between blade edges 604 within the same row 610.

It is also to be appreciated that the elastomeric laminate assembly operations herein may also be performed in conjunction with other operations. In some configurations, the elastomeric laminates 200 assembled with the methods and apparatuses herein may be subjected to various other manufacturing transformations before or after creating the low-stretch zones 402.

For example, as shown in Figure 8, the continuous elastomeric laminate 200 may advance to a slitting device 350, wherein the elastomeric laminate 200 is slit and separated along the machine direction MD into lanes, such as for example, a first continuous elastomeric laminate 200a and a second continuous elastomeric laminate 200b. It is to be appreciated that the elastomeric laminate 200 may be slit with a shear slitting operation or a crush slit operation. In a crush slit operation, the first substrate 204 and the second substrate 206 may be bonded together during the slitting operation. In some operations, the first and second substrates 204, 206 of an elastomeric laminate 200 may be bonded together along edges of the elastomeric laminate 200. For example, in some operations, edges of the first substrate 204 may be folded over opposing edge portions of the second substrate 206 to create sealed edges of the elastomeric laminate 200. It is to be appreciated that heat, pressure, adhesive, and/or ultrasonic bonding processes may be used to fixate such folded portions of the substrates. In some configurations, the locations of elastic strands 208 relative to side edges of elastomeric laminates 200 may be adjusted to change corrugation patterns along the side edges in desired manners.

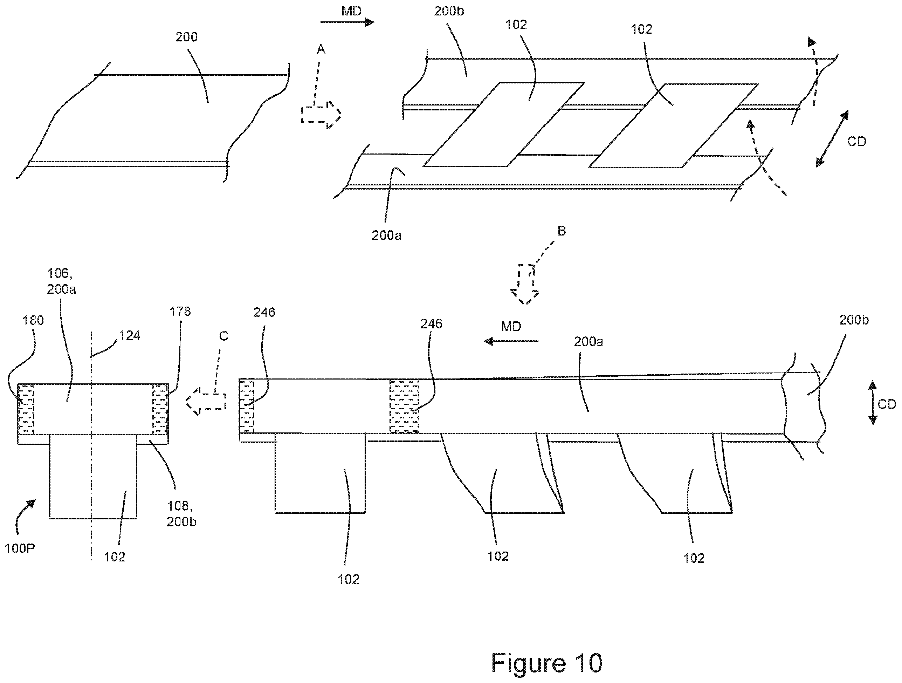

With continued reference to Figure 8, the slitting device 350 may be configured to cut the elastic laminate 200 in the machine direction MD through the low stretch zones 200 to create a first low-stretch zones 402a and second low-stretch zones 402b. It is to be appreciated that the first elastic laminate 200a may correspond with the first elastic belt 106 and the second elastic laminate 200b may correspond with the second elastic belt 108 described above. For example, as shown in Figure 10, when assembling diaper pants 100P, the elastic laminate 200 may be converted into a first elastic belt laminate 200a and/or a second elastic belt laminate 200b (represented by the dashed arrow "A"). The first elastic belt laminate 200a and the second elastic belt laminate 200b may be separated from each other in the cross direction CD. In turn, opposing end regions of chassis 102 may be connected with the low-stretch zones 402 in the first elastic belt laminate 200a and/or a second elastic belt laminate 200b. During subsequent assembly operations, the chassis 102 may be folded (represented by the dashed arrow "B") so as to position the first elastic belt laminate 200a into a facing relationship with the second elastic belt laminate 200b. Bonds 246 may be applied to the overlapping belt laminates 200a, 200b. Subsequently, discrete diaper pants 100P may be formed by separating the first and second belt laminates 200a, 200b into first and second belts 106, 108 by cutting along the cross direction CD through the first and second belt laminates 200a, 200b adjacent the bonds 246 (represented by the dashed arrow "C"). As such, the bonds 246 may be divided to define the first and second side seams 178, 180, respectively.

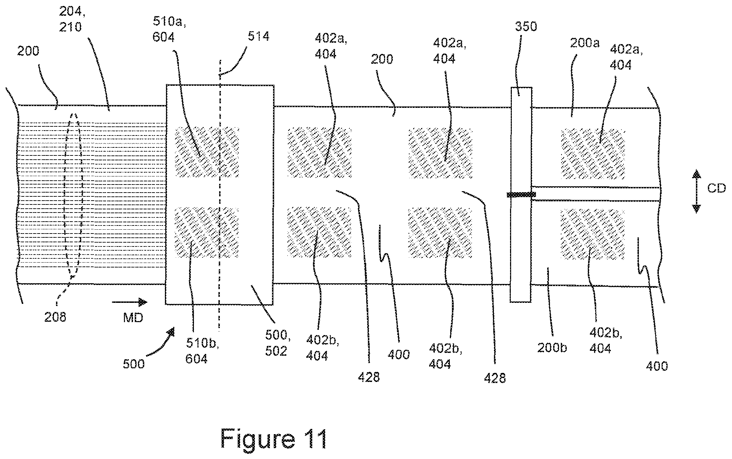

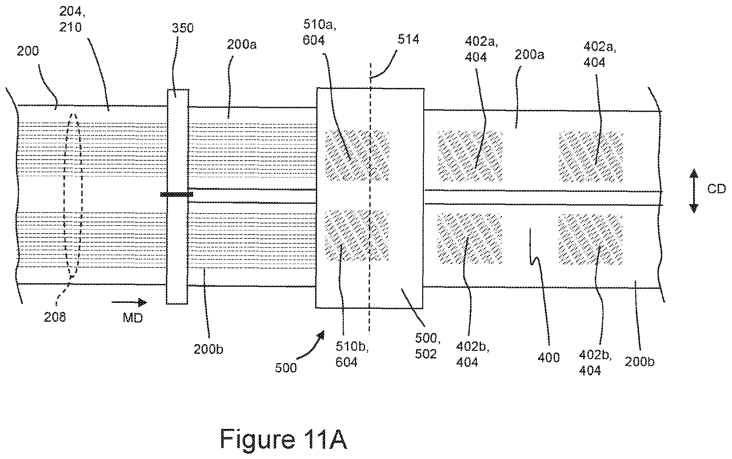

It is to be appreciated that the cutting device 500 may be configured in various ways to create low-stretch zones having various orientations and positions on the elastic laminate 200. In some configurations, the low-stretch zones 402 may be positioned on the elastic laminate 200 to help prevent cut lines 404 from being positioned on edges of elastic laminates 200 to hep reduce or prevent edge fraying that may be caused by the cut lines 404. For example, as shown in Figure 11, the knife roll 502 may be configured with a first group of blades 510a separated in the cross direction CD from a second group of blades 510b. As such, the cutting device 500 may be configured to create first low-stretch zones 402a and second low-stretch zones 402b in the elastic laminate 200 before advancing to the slitting device 350. In turn, first low-stretch zones 402a and second low-stretch zones 402b on the elastic laminate 200 may be separated from each other in the cross direction CD by a gap region 428. The slitting device 350 may be configured to cut the elastic laminate 200 in the machine direction MD through the gap regions 428 to create the first elastic laminate 200a with the first low-stretch zones 402a and the second elastic laminate 200b with the second low-stretch zones 402b. In some configurations, the first low-stretch zones 402a and the second elastic laminate 200b may be spaced from and not positioned directly on inboard edges of the first elastic laminate 200a and the second elastic laminate 200b created by the slitting device 350. In some configurations, the slitting device 350 may be arranged upstream of the cutting device 500 such as shown in Figure 11A.

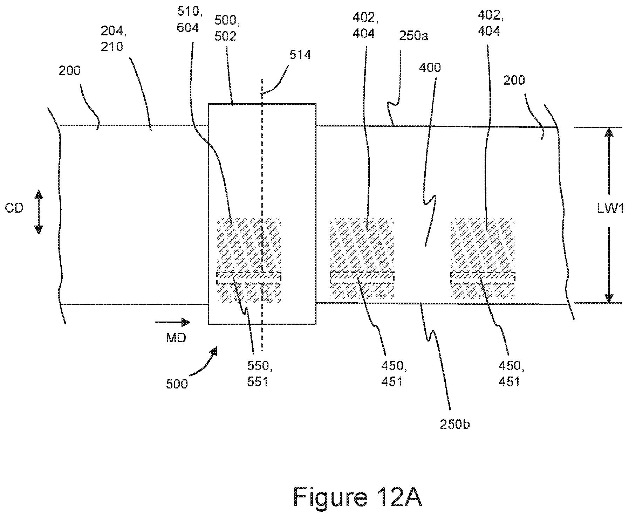

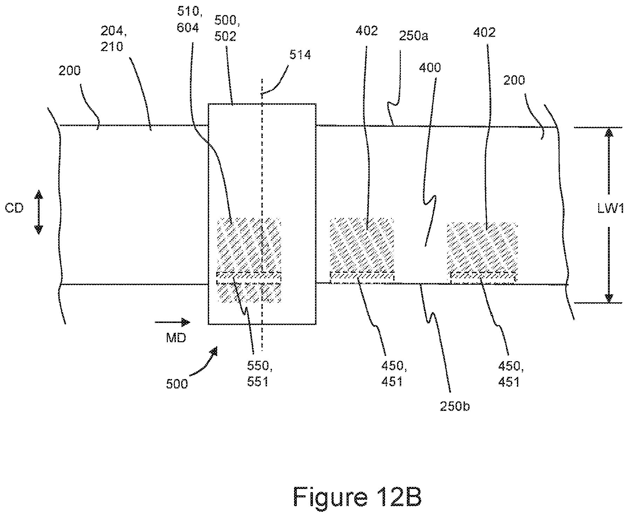

In some configurations, the knife roll 502 may include a group of blades 510 adapted to create low-stretch zones 402 in elastic laminates 200 having different widths in the cross direction CD without having to change the knife 502 to accommodate the different widths. For example, as shown in Figures 12A and 12B, the knife roll 502 may include a group of blades 510 with a pattern gap region 550. The pattern gap region 550 may be defined by a region with blades 551 that may have different attributes than the blades 510 outside the pattern gap region 550. For example, there may be less quantities of blades 551 spaced along the circumferential and axial directions in the pattern gap 550 than blades 510 outside the pattern gap region 550. In another example, some blades 551 in the pattern gap 550 may comprise radial heights that are less than the blades 510 outside the pattern gap region 550. As such, the knife roll 502 creates low-stretch zones 402 with gap regions 450 with cut lines 404 that correspond with the blades 551 in the pattern gap region 550. Although the elastic strands 208 are cut into discrete pieces in the gap region 450, the low-stretch zone 402 includes a relatively lower quantity of cut lines 404 per unit area of the elastic laminate 200 in the gap region 450 than a quantity of cut lines 404 per unit area of the elastic laminate 200 outside the gap region 450.

As shown in Figures 12A and 12B, the same knife roll 500 can be used to create low-stretch zones 402 in elastic laminates 200 having different cross directional widths defined by distances between opposing first and second edges 250a, 250b of the elastic laminate 200. For example, the elastic laminate 200 in Figure 12A may define a width LW1 in the cross direction CD, and the elastic laminate 200 in Figure 12B may define a width LW2 in the cross direction CD, wherein LW2 is less than LW1. As shown in Figure 12A, the second edge 250b of the elastic laminate 200 may be positioned so as to advance adjacent to or outboard of the group of blades 510. As such, relatively few or no cut lines 404 may be present on the second edge 250b. As shown in Figure 12B, the second edge 250b of the elastic laminate 200 may be positioned so as to advance through the pattern gap region 550 of the group of blades 510. As such, relatively few cut lines 404 may be present on the second edge 250b.

In some configurations, the rotational speeds of the knife roll 502 may be adjusted to create different machine direction MD lengths of low-stretch zones 402 in the elastic laminate 200. For example, the knife roll 502 rotating at a first rotational speed may create a low-stretch zone 402 having a first length in the machine direction MD in an elastic laminate 200 advancing at a first speed. In turn, the same knife roll 502 rotating at a second rotational speed higher than the first rotational speed may create a low-stretch zone 402 having a second length in the machine direction MD that is less than the first length in an elastic laminate 200 advancing at the same first speed.

It is to be appreciated that the absorbent articles 100 herein may be configured to include a disposal feature, such as disclosed in Patent Publication Nos. US 7867208 B and EP 3716929 B, which are incorporated by reference herein. In some forms, the disposal feature may comprise a disposal tape that may be bonded with the first elastic belt 106 or the second elastic belt 108. The disposal tape may be secured to a diaper pant in a folded configuration. In use, an end portion of disposal tape may be pulled in a direction away from a soiled diaper to extend the disposal tape from the folded configuration. The soiled diaper may then be rolled-up onto itself and the extended disposal tape may be used to help secure the soiled diaper in a rolled-up configuration. In some configurations, such a disposal tape may be bonded to the first elastic belt 106 or the second elastic belt 108 partially or completely within a low stretch zone 402. However, a relatively high concentration of cut lines 404 located in a region where the disposal is bonded with the first elastic belt 106 or the second elastic belt 108 may result in a reduced bond strength between the disposal tape and the belt. As such, in some configurations, the low stretch zone 402 may include a disposal tape bond region 401 that helps provide a desired bond strength between the disposal tape and belt. In turn, the bond strength may be maintained to be high enough to withstand the forces exerted on the disposal tape bond region 401 when pulling the disposal tape from the folded to the extended configuration as well as when holding the diaper pant in a rolled-up configuration. As discussed in more detail below, the disposal tape bond region 401 may be configured with a relatively lower concentration of cut lines 404 than exist in the remainder of the low stretch zone 402. Such varying concentration of cut lines 404 may be created by corresponding variations in concentrations of blades 510 on a cutting roll 502.

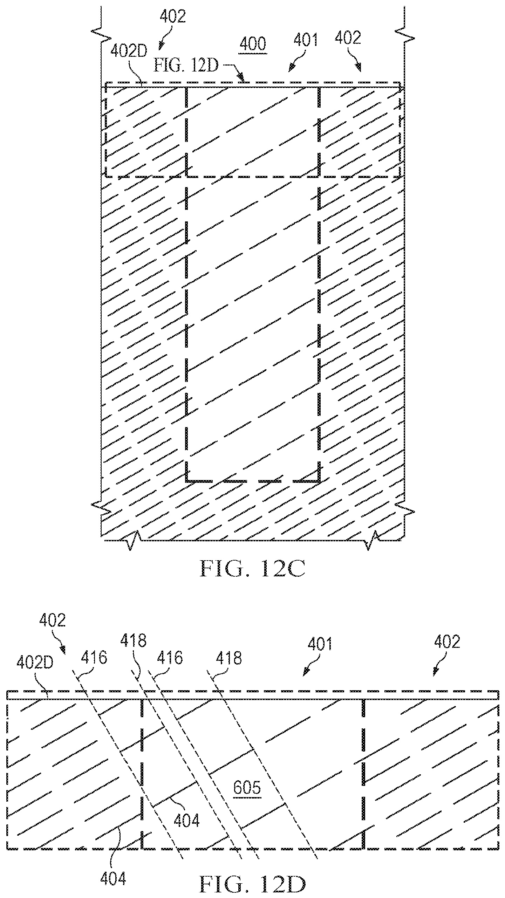

Referring to Figure 12C, the disposal tape bond region 401 may be provided generally along the longitudinal axis and towards the distal end line 402D of the low stretch zone 402, which disposal tape bond region 401 may have a different cut-line concentration as the low stretch zone 402. Providing such disposal tape bond region 401 may be beneficial when the absorbent article is assembled with a disposal tape. When the low stretch zone 402 has a first cut-line concentration and the disposal tape bond region 401 has a second cut-line concentration, the second cut-line concentration is from about 30% to about 60% of the first cut-line concentration. The lower concentration of cut lines in the disposal tape bond region 401 may provide a higher bond strength of the elastic laminate 200 compared to that of the low stretch zone 402, while still providing the disposal tape bond region 401 with lower elasticity than the high stretch zone 400.

Referring to Figure 12D, the cut-line concentration of the disposal tape bond region 401 may be adjusted by thinning blades 510 on a cutting roll 502, wherein the blades 510 for providing the disposal tape bond region 401 are aligned in the direction of the blades 510 for providing the low stretch zone 402. For example, to create a second cut-line concentration of 50% compared to the first cut-line concentration, every other blade may be thinned, or every 2 out of 4 blades may be thinned. For example, to create a second cut-line concentration of 40% compared to the first cut-line concentration, every 3 out of 5 blades may be thinned. For example, to create a second cut-line concentration of 33.3% compared to the first cut-line concentration, one of every 3 blades may be thinned. Regardless of the difference between the first and second cut-line concentrations, the thinning of the blades 510 may be adjusted so that the remaining blades 510 are aligned in the direction of the blades 510 for providing the low stretch zone 402. Further, the non-cut area 605 created in a region matching the thinned blades may be adjusted so that the distance between any cut line in the disposal tape bond region 401, in the direction of the reference line, is no more than about 8mm.

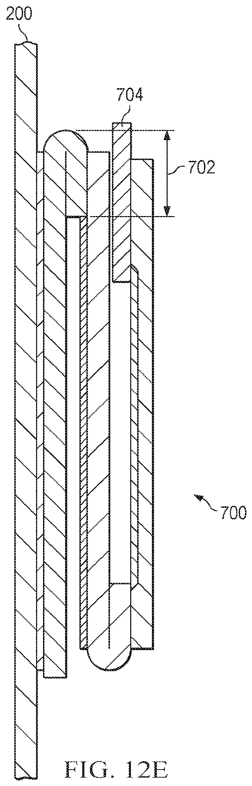

Figure 12E shows an example of a disposal tape 700 that may be bonded to the elastic laminate 200 generally along the longitudinal axis. The disposal tape 700 having a certain longitudinal dimension, the disposal tape 700 may be bonded partially or completely within a low stretch zone 402, namely the bonding may exist beyond the distal end line 402D of the low stretch zone 402. When a portion of the disposal tape 700 is bonded beyond the distal end line 402D of the low stretch zone 402, the area of the elastic laminate 200 overlapping the disposal tape 700 may be provided a disposal tape bond region 401. The disposal tape 700 may have a certain length folded into 2 or 3 parts, wherein the disposal tape 700 may be lifted from the folded position by first releasing the tab 704 from the remainder of the disposal tape 700 and pulling away from the elastic laminate 200. Referring to Figure 12E, an example of a disposal tape folded into 3 parts is depicted. The layer of the disposal tape 700 directly bonded to the elastic laminate 200 has a distal end 702. The distal end 702 of the disposal tape is the portion receiving the greatest stress when the disposal tape 700 is stretched into its full length for disposal. The distal end 702 may overlap the high stretch zone 400, while the remainder of the disposal tape overlaps the disposal tape bond region 401.

By arranging the blades for providing the disposal tape bond region 401 in the manner discussed above with reference to Figures 12B and 12C for example, the elastic materials in the disposal tape bond region 401 are cut, while the bond strength of the elastic laminate 200 in the disposal tape bond region 401 may be secured to endure the peeling strength when the disposal tape 700 is pulled away from the elastic laminate 200.

AVERAGE DECITEX (AVERAGE-DTEX)

The Average Decitex Method is used to calculate the Average-Dtex on a length-weighted basis for elastic fibers present in an entire article, or in a specimen of interest extracted from an article. The decitex value is the mass in grams of a fiber present in 10,000 meters of that material in the relaxed state. The decitex value of elastic fibers or elastic laminates containing elastic fibers is often reported by manufacturers as part of a specification for an elastic fiber or an elastic laminate including elastic fibers. The Average-Dtex is to be calculated from these specifications if available. Alternatively, if these specified values are not known, the decitex value of an individual elastic fiber is measured by determining the cross-sectional area of a fiber in a relaxed state via a suitable microscopy technique such as scanning electron microscopy (SEM), determining the composition of the fiber via Fourier Transform Infrared (FT-IR) spectroscopy, and then using a literature value for density of the composition to calculate the mass in grams of the fiber present in 10,000 meters of the fiber. The manufacturer-provided or experimentally measured decitex values for the individual elastic fibers removed from an entire article, or specimen extracted from an article, are used in the expression below in which the length-weighted average of decitex value among elastic fibers present is determined.

The lengths of elastic fibers present in an article or specimen extracted from an article is calculated from overall dimensions of and the elastic fiber pre-strain ratio associated with components of the article with these or the specimen, respectively, if known. Alternatively, dimensions and/or elastic fiber pre-strain ratios are not known, an absorbent article or specimen extracted from an absorbent article is disassembled and all elastic fibers are removed. This disassembly can be done, for example, with gentle heating to soften adhesives, with a cryogenic spray (e.g., Quick-Freeze, Miller-Stephenson Company, Danbury, CT), or with an appropriate solvent that will remove adhesive but not swell, alter, or destroy elastic fibers. The length of each elastic fiber in its relaxed state is measured and recorded in millimeters (mm) to the nearest mm.

Calculation of Average-Dtex

For each of the individual elastic fibers $f_i$ of relaxed length $L_i$ and fiber decitex value $d_i$ (obtained either from the manufacturer's specifications or measured experimentally) present in an absorbent article, or specimen extracted from an absorbent article, the Average-Dtex for that absorbent article or specimen extracted from an absorbent article is defined as:

$$\text{Average-Dtex} = \frac{\sum_{i=1}^{n}(L_i \times d_i)}{\sum_{i=1}^{n} L_i}$$

where $n$ is the total number of elastic fibers present in an absorbent article or specimen extracted from an absorbent article. The Average-Dtex is reported to the nearest integer value of decitex (grams per 10 000 m).
If the decitex value of any individual fiber is not known from specifications, it is experimentally determined as described below, and the resulting fiber decitex value(s) are used in the above equation to determine Average-Dtex.

Experimental Determination of Decitex Value for a Fiber

For each of the elastic fibers removed from an absorbent article or specimen extracted from an absorbent article according to the procedure described above, the length of each elastic fiber $L_k$ in its relaxed state is measured and recorded in millimeters (mm) to the nearest mm. Each elastic fiber is analyzed via FT-IR spectroscopy to determine its composition, and its density $\rho_k$ is determined from available literature values. Finally, each fiber is analyzed via SEM. The fiber is cut in three approximately equal locations perpendicularly along its length with a sharp blade to create a clean cross-section for SEM analysis. Three fiber segments with these cross sections exposed are mounted on an SEM sample holder in a relaxed state, sputter coated with gold, introduced into an SEM for analysis, and imaged at a resolution sufficient to clearly elucidate fiber cross sections. Fiber cross sections are oriented as perpendicular as possible to the detector to minimize any oblique distortion in the measured cross sections. Fiber cross sections may vary in shape, and some fibers may consist of a plurality of individual filaments. Regardless, the area of each of the three fiber cross sections is determined (for example, using diameters for round fibers, major and minor axes for elliptical fibers, and image analysis for more complicated shapes), and the average of the three areas $a_k$ for the elastic fiber, in units of micrometers squared ($\mu m^2$), is recorded to the nearest 0.1 $\mu m^2$. The decitex $d_k$ of the kth elastic fiber measured is calculated by:

$$d_k = 10\ 000\ \text{m} \times a_k \times \rho_k \times 10^{-6}$$

where $d_k$ is in units of grams (per calculated 10,000 meter length), $a_k$ is in units of $\mu m^2$, and $\rho_k$ is in units of grams per cubic centimeter (g/cm$^3$). For any elastic fiber analyzed, the experimentally determined $L_k$ and $d_k$ values are subsequently used in the expression above for Average-Dtex.

AVERAGE-STRAND-SPACING

Using a ruler calibrated against a certified NIST ruler and accurate to 0.5 mm, measure the distance between the two distal strands within a section to the nearest 0.5 mm, and then divide by the number of strands in that section - 1

$$\text{Average-Strand-Spacing} = d/(n\text{-}1) \text{ where n>1}$$

report to the nearest 0.1 mm.

AVERAGE-PRE-STRAIN

The Average-Pre-Strain of a specimen are measured on a constant rate of extension tensile tester (a suitable instrument is the MTS Insight using Testworks 4.0 Software, as available from MTS Systems Corp., Eden Prairie, MN) using a load cell for which the forces measured are within 1% to 90% of the limit of the cell. Articles are conditioned at 23 °C $\pm$ 2 C° and 50% $\pm$ 2% relative humidity for 2 hours prior to analysis and then tested under the same environmental conditions.

Program the tensile tester to perform an elongation to break after an initial gage length adjustment. First raise the cross head at 10 mm/min up to a force of 0.05N. Set the current gage to the adjusted gage length. Raise the crosshead at a rate of 100 mm/min until the specimen breaks (force drops 20% after maximum peak force). Return the cross head to its original position. Force and extension data is acquired at a rate of 100 Hz throughout the experiment.

Set the nominal gage length to 40 mm using a calibrated caliper block and zero the crosshead. Insert the specimen into the upper grip such that the middle of the test strip is positioned 20 mm below the grip. The specimen may be folded perpendicular to the pull axis, and placed in the grip to achieve this position. After the grip is closed the excess material can be trimmed. Insert the specimen into the lower grips and close. Once again, the strip can be folded, and then trimmed after the grip is closed. Zero the load cell. The specimen should have a minimal slack but less than 0.05 N of force on the load cell. Start the test program.

From the data construct a Force (N) verses Extension (mm). The Average-Pre-Strain is calculated from the bend in the curve corresponding to the extension at which the nonwovens in the elastic are engaged. Plot two lines, corresponding to the region of the curve before the bend (primarily the elastics), and the region after the bend (primarily the nonwovens). Read the extension at which these two lines intersect, and calculate the % Pre-Strain from the extension and the corrected gage length. Record as % Pre-strain 0.1%. Calculate the arithmetic mean of three replicate samples for each elastomeric laminate and Average-Pre-Strain to the nearest 0.1%.

Bio-Based Content for Components

Components of the absorbent articles described herein may at least partially be comprised of bio-based content as described in U.S. Pat. Appl. No. 2007/0219521A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and 2016/0206774, and U.S. Pat. No. 9,169,366. Example bio-based polyolefins for use in the present disclosure comprise polymers available under the designations SHA7260™ SHE150™, or SGM9450F™ (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

Recycle Friendly and Bio-Based Absorbent Articles

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in US2019/0192723, published on June 27, 2019.

**Claims**

1. An absorbent article comprising:

   a body facing surface (132) and a garment facing surface (134);
   a front waist region (116) and a back waist region (118), the back waist region (118) separated from the front waist region (116) by a crotch region (119), the front waist region (116) comprising a front waist edge (121), and the back waist region comprising (118) a back waist edge (122), wherein a longitudinal axis (124) extends perpendicularly through the front waist edge (121) and the back waist edge (122), and wherein a lateral axis (126) extends perpendicularly to the longitudinal axis;
   an absorbent assembly (140) extending longitudinally through the crotch region (119) between the front waist region (116) and the back waist region (118), the absorbent assembly (140) positioned between the body facing surface (132) and the garment facing surface (134);
   wherein at least one of the front waist region (116) and the back waist region (118) comprises:

   an elastic material (167) positioned between and connected with a first substrate (162) and a second substrate (164);
   a first high-stretch zone (400a) and a second high-stretch zone (400b) separated laterally by a low-stretch zone (402), wherein the first and second high-stretch zones (400a, 400b) are elasticated by the elastic material (167);
   wherein the low-stretch zone (402) comprises cut lines (404) separating the elastic material (167) into first discrete pieces (406a) and second discrete pieces (406b);
   wherein the first discrete pieces (406a) of elastic material (167) comprise a first length and wherein the second discrete pieces (406b) of elastic material (167) comprise a second length, wherein the second length is greater than the first length; and

   wherein each cut line (404) is oriented to define an offset angle relative to the lateral axis (126) that is greater than 0 degrees and less than 45 degrees.

2. The absorbent article according to claim 1, wherein the cut lines (404) penetrate through the elastic material (167), the first substrate (162), and the second substrate (164).

3. The absorbent article according to claim 1 or 2, wherein the wherein the cut lines (404) are arranged in rows (410) comprising at least a first row (410a) and a second row (410b) neighboring the first row (410a), wherein the first length of the first discrete pieces (406a) of elastic material (167) is defined by a distance extending laterally between neighboring cut lines (404) in the first row (410a), and wherein the second length of the second discrete pieces (406b) of elastic material (167) is defined by a distance extending laterally between cut lines (404) in the first row (410a) and cut lines (404) in the second row (410b).

4. The absorbent article according to claim 3, wherein cut lines (404) in the first row (410a) and the second row (410b) extend for a length from a first end (404c) to a second end (404d); and wherein cut lines (404) in the first row (410a) and cut lines (404) in the second row (410b) are parallel to each other and wherein the first ends (404c) of cut lines (404) in the first row (410a) and the first ends (404c) of cut lines (404) in the second row (410b) are aligned along first reference lines (416) that are oriented to define a row angle relative to the lateral axis (126) that is less than 90 degrees and greater than 45 degrees.

5. The absorbent article according to claim 3 or 4, wherein the second ends (404d) of cut lines (404) in the first row (410a) and the second ends (404d) of cut lines (404) in the second row (410b) are aligned along second reference lines (418), wherein the second reference lines (418) are parallel to the first reference lines (416).

6. The absorbent article according to claim 5, wherein the second reference line (418) of the first row (410a) is separated from the first reference line (416) of the second row (410b) by a row gap distance (422), wherein the row gap distance (422) is about 1.9 mm.

7. The absorbent article according to any of claims 3-6, wherein the length of each cut line (404) in the first row (410a) and the second row (410b) is about 4 mm, and wherein the cut lines (404) in the first row (410a) are separated from each other by a cut line gap distance (414), and wherein the cut line gap distance (414) is about 1.9 mm.

8. The absorbent article according to any of the preceding claims, wherein the elastic material (167) comprises elastic strands (168) and wherein the first discrete pieces (406a) of elastic material (167) comprise first discrete pieces of elastic strands (168) and wherein the second discrete pieces (406b) of elastic material (167) comprise second discrete pieces of elastic strands (168), wherein the elastic strands (168), the first discrete pieces of elastic strands (168), and the second discrete pieces of elastic strands (168) are continuously bonded with at least one of the first substrate (162) and the second substrate (164).

9. The absorbent article according to claim 8, wherein the elastic strands (168), the first discrete pieces of elastic strands (168), and the second discrete pieces of elastic strands (168) are bonded with adhesive applied to at least one of the first substrate (162), the second substrate (164), and the elastic strands (168).

10. The absorbent article according to any of the preceding claims, wherein a ratio of the second length to the first length is about 2:1.

11. The absorbent article according to any of the preceding claims, wherein the low stretch zone (402) comprises a disposal tape bond region (401), wherein the low stretch zone (402) comprises a first cut-line concentration and the disposal tape bond region (401) comprises a second cut-line concentration, wherein the second cut-line concentration is from about 30% to about 60% of the first cut-line concentration.

12. The absorbent article of claim 11, wherein the cut-line concentration of the disposal tape bond region (401) is adjusted by thinning blades, wherein the blades for providing the disposal tape bond region (401) are aligned in a same direction of blades for providing the low stretch zone (402).

13. The absorbent article of claim 12, wherein a distance between any cut line (404) in the disposal tape bond region (401) is no more than about 8mm.

14. The absorbent article of claim 11, comprising a disposal tape (700) joined to the front waist region (116) or the back waist region (118), wherein at least a portion of the disposal tape (700) overlaps the disposal tape bond region (401).

15. The absorbent article of claim 14, wherein the disposal tape (701) comprises a distal end (702), wherein the distal end (702) overlaps a high stretch zone (400).

**Patentansprüche**

1. Absorptionsartikel, umfassend:

eine körperseitige Oberfläche (132) und eine bekleidungsseitige Oberfläche (134);

einen vorderseitigen Taillenbereich (116) und einen rückseitigen Taillenbereich (118), wobei der rückseitige Taillenbereich (118) von dem vorderseitigen Taillenbereich (116) durch einen Schrittbereich (119) getrennt ist, der vorderseitige Taillenbereich (116) umfassend einen vorderseitigen Taillenrand (121) und der rückseitige Taillenbereich (118) umfassend einen rückseitigen Taillenrand (122), wobei sich eine Längsachse (124) lotrecht durch den vorderseitigen Taillenrand (121) und den rückseitigen Taillenrand (122) erstreckt und wobei sich eine Querachse (126) lotrecht zu der Längsachse erstreckt;

eine Absorptionseinheit (140), die sich in Längsrichtung durch den Schrittbereich (119) zwischen dem vorderseitigen Taillenbereich (116) und dem rückseitigen Taillenbereich (118) erstreckt, wobei die Absorptionseinheit (140) zwischen der körperseitigen Oberfläche (132) und der bekleidungsseitigen Oberfläche (134) angeordnet ist;

wobei wenigstens einer des vorderseitigen Taillenbereichs (116) und des rückseitigen Taillenbereichs (118) umfasst:

ein elastisches Material (167), das zwischen einem ersten Substrat (162) und einem zweiten Substrat (164) angeordnet und mit diesen verbunden ist;

einen ersten Hochdehnungsbereich (400a) und einen zweite Hochdehnungsbereich (400b), die durch einen Niedrigdehnungsbereich (402) seitlich getrennt sind, wobei der erste und der zweite Hochdehnungsbereich (400a, 400b) durch das elastische Material (167) elastifiziert sind;

wobei der Niedrigdehnungsbereich (402) Ausschnittlinien (404) umfasst, die das elastische Material (167) in erste separate Stücke (406a) und zweite separate Stücke (406b) trennen;

wobei die ersten separaten Stücke (406a) aus elastischem Material (167) eine erste Länge umfassen und wobei die zweiten separaten Stücke (406b) aus elastischem Material (167) eine zweite Länge umfassen, wobei die zweite Länge größer als die erste Länge ist; und

wobei jede Ausschnittlinie (404) ausgerichtet ist, um einen Versatzwinkel relativ zu der Querachse (126) zu definieren, der größer als 0 Grad und kleiner als 45 Grad ist.

2.  Absorptionsartikel nach Anspruch 1, wobei die Ausschnittlinien (404) durch das elastische Material (167), das erste Substrat (162) und das zweite Substrat (164) hindurchgehen.

3.  Absorptionsartikel nach Anspruch 1 oder 2, wobei die Ausschnittlinien (404) in Reihen (410) angeordnet sind, umfassend wenigstens eine erste Reihe (410a) und eine zweite Reihe (410b), die benachbart zu der ersten Reihe (410a) ist, wobei die erste Länge der ersten separaten Stücke (406a) aus elastischem Material (167) durch einen Abstand definiert ist, der sich seitlich zwischen benachbarten Ausschnittlinien (404) in der ersten Reihe (410a) erstreckt, und wobei die zweite Länge der zweiten separaten Stücke (406b) aus elastischem Material (167) durch einen Abstand definiert ist, der sich seitlich zwischen Ausschnittlinien (404) in der ersten Reihe (410a) und Ausschnittlinien (404) in der zweiten Reihe (410b) erstreckt.

4.  Absorptionsartikel nach Anspruch 3, wobei Ausschnittlinien (404) in der ersten Reihe (410a) und der zweiten Reihe (410b) sich über eine Länge von einem ersten Ende (404c) zu einem zweiten Ende (404d) erstrecken; und wobei Ausschnittlinien (404) in der ersten Reihe (410a) und Ausschnittlinien (404) in der zweiten Reihe (410b) parallel zueinander sind und wobei die ersten Enden (404c) der Ausschnittlinien (404) in der ersten Reihe (410a) und die ersten Enden (404c) der Ausschnittlinien (404) in der zweiten Reihe (410b) entlang erster Referenzlinien (416) gerichtet sind, die ausgerichtet sind, um einen Reihenwinkel relativ zu der Querachse (126) zu definieren, der kleiner als 90 Grad und größer als 45 Grad ist.

5.  Absorptionsartikel nach Anspruch 3 oder 4, wobei die zweiten Enden (404d) der Ausschnittlinien (404) in der ersten Reihe (410a) und die zweiten Enden (404d) der Ausschnittlinien (404) in der zweiten Reihe (410b) entlang zweiter Referenzlinien (418) gerichtet sind, wobei die zweiten Referenzlinien (418) parallel zu den ersten Referenzlinien (416) sind.

6.  Absorptionsartikel nach Anspruch 5, wobei die zweite Referenzlinie (418) der ersten Reihe (410a) von der ersten Referenzlinie (416) der zweiten Reihe (410b) durch einen Reihenspalt (422) getrennt ist, wobei der Reihenspalt (422) etwa 1,9 mm beträgt.

7.  Absorptionsartikel nach einem der Ansprüche 3 bis 6, wobei die Länge jeder Ausschnittlinie (404) in der ersten Reihe (410a) und der zweiten Reihe (410b) etwa 4 mm beträgt und wobei die Ausschnittlinien (404) in der ersten Reihe (410a) durch einen Ausschnittlinienspaltabstand (414) voneinander getrennt sind und wobei der Ausschnittlinienspaltabstand (414) etwa 1,9 mm beträgt.

8. Absorptionsartikel nach einem der vorstehenden Ansprüche, wobei das elastische Material (167) elastische Stränge (168) umfasst und wobei die ersten separaten Stücke (406a) des elastischen Materials (167) erste separate Stücke elastischer Stränge (168) umfassen und wobei die zweiten separaten Stücke (406b) des elastischen Materials (167) zweite separate Stücke elastischer Stränge (168) umfassen, wobei die elastischen Stränge (168), die ersten separaten Stücke elastischer Stränge (168) und die zweiten separaten Stücke elastischer Stränge (168) ununterbrochen mit wenigstens einem des ersten Substrats (162) und des zweiten Substrats (164) gebunden sind.

9. Absorptionsartikel nach Anspruch 8, wobei die elastischen Stränge (168), die ersten separaten Stücke elastischer Stränge (168) und die zweiten separaten Stücke elastischer Stränge (168) mit Klebstoff gebunden sind, der auf wenigstens eines des ersten Substrats (162), des zweiten Substrats (164) und der elastischen Stränge (168) aufgetragen ist.

10. Absorptionsartikel nach einem der vorstehenden Ansprüche, wobei ein Verhältnis der zweiten Länge zu der ersten Länge etwa 2 : 1 beträgt.

11. Absorptionsartikel nach einem der vorstehenden Ansprüche, wobei der Niedrigdehnungsbereich (402) einen Einwegbandbindungsbereich (401) umfasst, wobei der Niedrigdehnungsbereich (402) eine erste Ausschnittlinienkonzentration umfasst und der Einwegbandbindungsbereich (401) eine zweite Ausschnittlinienkonzentration umfasst, wobei die zweite Ausschnittlinienkonzentration etwa 30 % bis etwa 60 % der ersten Ausschnittlinienkonzentration beträgt.

12. Absorptionsartikel nach Anspruch 11, wobei die Ausschnittlinienkonzentration des Einwegbandbindungsbereichs (401) durch Ausdünnungslamellen angepasst wird, wobei die Lamellen zum Bereitstellen des Einwegbandbindungsbereichs (401) in eine gleiche Lamellenrichtung zum Bereitstellen des Niedrigdehnungsbereichs (402) gerichtet sind.

13. Absorptionsartikel nach Anspruch 12, wobei der Abstand zwischen den Ausschnittlinien (404) in dem Einwegbandbindungsbereich (401) nicht mehr als etwa 8 mm beträgt.

14. Absorptionsartikel nach Anspruch 11, umfassend ein Einwegband (700), das mit dem vorderseitigen Taillenbereich (116) oder dem rückseitigen Taillenbereich (118) gefaltet ist, wobei wenigstens einen Teil des Einwegbands (700) den Einwegbandbindungsbereich (401) überlappt.

15. Absorptionsartikel nach Anspruch 14, wobei das Entsorgungsband (701) ein distales Ende (702) umfasst, wobei das distale Ende (702) einen Hochdehnungsbereich (400) überlappt.

**Revendications**

1. Article absorbant comprenant :

une surface tournée vers le corps (132) et une surface tournée vers le vêtement (134) ;
une région de ceinture avant (116) et une région de ceinture arrière (118), la région de ceinture arrière (118) étant séparée de la région de ceinture avant (116) par une région d'entrejambe (119), la région de ceinture avant (116) comprenant un bord de ceinture avant (121), et la région de ceinture arrière comprenant (118) un bord de ceinture arrière (122), dans lequel un axe longitudinal (124) s'étend perpendiculairement à travers le bord de ceinture avant (121) et le bord de ceinture arrière (122), et dans lequel un axe latéral (126) s'étend perpendiculairement à l'axe longitudinal ;
un ensemble absorbant (140) s'étendant longitudinalement à travers la région d'entrejambe (119) entre la région de ceinture avant (116) et la région de ceinture arrière (118), l'ensemble absorbant (140) étant positionné entre la surface tournée vers le corps (132) et la surface tournée vers le vêtement (134) ;
dans lequel au moins l'une parmi la région de ceinture avant (116) et la région de ceinture arrière (118) comprend :

un matériau élastique (167) positionné entre et relié à un premier substrat (162) et un second substrat (164) ;
une première zone de fort allongement (400a) et une seconde zone de fort allongement (400b) séparées latéralement par une zone de faible allongement (402), dans lequel les première et seconde zones de fort allongement (400a, 400b) sont rendues élastiques par le matériau élastique (167) ;

dans lequel la zone de faible allongement (402) comprend des lignes de coupe (404) séparant le matériau élastique (167) en premiers morceaux distincts (406a) et en seconds morceaux distincts (406b) ;
dans lequel les premiers morceaux distincts (406a) de matériau élastique (167) comprennent une première longueur et dans lequel les seconds morceaux distincts (406b) de matériau élastique (167) comprennent une seconde longueur, dans lequel la seconde longueur est supérieure à la première longueur ; et
dans lequel chaque ligne de coupe (404) est orientée pour définir un angle de décalage par rapport à l'axe latéral (126) qui est supérieur à 0 degré et inférieur à 45 degrés.

2. Article absorbant selon la revendication 1, dans lequel les lignes de coupe (404) pénètrent à travers le matériau élastique (167), le premier substrat (162), et le second substrat (164).

3. Article absorbant selon la revendication 1 ou 2, dans lequel les lignes de coupe (404) sont agencées en rangées (410) comprenant au moins une première rangée (410a) et une seconde rangée (410b) voisine de la première rangée (410a), dans lequel la première longueur des premiers morceaux distincts (406a) de matériau élastique (167) est définie par une distance s'étendant latéralement entre des lignes de coupe (404) voisines dans la première rangée (410a), et dans lequel la seconde longueur des seconds morceaux distincts (406b) de matériau élastique (167) est définie par une distance s'étendant latéralement entre des lignes de coupe (404) dans la première rangée (410a) et des lignes de coupe (404) dans la seconde rangée (410b).

4. Article absorbant selon la revendication 3, dans lequel des lignes de coupe (404) dans la première rangée (410a) et la seconde rangée (410b) s'étendent sur une longueur allant d'une première extrémité (404c) à une seconde extrémité (404d) ; et dans lequel des lignes de coupe (404) de la première rangée (410a) et des lignes de coupe (404) de la seconde rangée (410b) sont parallèles les unes aux autres et dans lequel les premières extrémités (404c) de lignes de coupe (404) de la première rangée (410a) et les premières extrémités (404c) de lignes de coupe (404) de la seconde rangée (410b) sont alignées le long de premières lignes de référence (416) qui sont orientées pour définir un angle de rangée par rapport à l'axe latéral (126) qui est inférieur à 90 degrés et supérieur à 45 degrés.

5. Article absorbant selon la revendication 3 ou 4, dans lequel les secondes extrémités (404d) de lignes de coupe (404) dans la première rangée (410a) et les secondes extrémités (404d) de lignes de coupe (404) dans la seconde rangée (410b) sont alignées le long de secondes lignes de référence (418), dans lequel les secondes lignes de référence (418) sont parallèles aux premières lignes de référence (416).

6. Article absorbant selon la revendication 5, dans lequel la seconde ligne de référence (418) de la première rangée (410a) est séparée de la première ligne de référence (416) de la seconde rangée (410b) par une distance d'espacement de rangée (422), dans lequel la distance d'espacement de rangée (422) est d'environ 1,9 mm.

7. Article absorbant selon l'une quelconque des revendications 3 à 6, dans lequel la longueur de chaque ligne de coupe (404) dans la première rangée (410a) et la seconde rangée (410b) est d'environ 4 mm, et dans lequel les lignes de coupe (404) dans la première rangée (410a) sont séparées les unes des autres par une distance d'espacement de ligne de coupe (414), et dans lequel la distance d'espacement de ligne de coupe (414) est d'environ 1,9 mm.

8. Article absorbant selon l'une quelconque des revendications précédentes, dans lequel le matériau élastique (167) comprend des brins élastiques (168) et dans lequel les premiers morceaux distincts (406a) de matériau élastique (167) comprennent des premiers morceaux distincts de brins élastiques (168) et dans lequel les seconds morceaux distincts (406b) de matériau élastique (167) comprennent des seconds morceaux distincts de brins élastiques (168), dans lequel les brins élastiques (168), les premiers morceaux distincts de brins élastiques (168), et les seconds morceaux distincts de brins élastiques (168) sont liés de manière continue à au moins l'un parmi le premier substrat (162) et le second substrat (164).

9. Article absorbant selon la revendication 8, dans lequel les brins élastiques (168), les premiers morceaux distincts de brins élastiques (168), et les seconds morceaux distincts de brins élastiques (168) sont liés avec un adhésif appliqué à au moins l'un parmi le premier substrat (162), le second substrat (164), et les brins élastiques (168).

10. Article absorbant selon l'une quelconque des revendications précédentes, dans lequel le rapport entre la seconde longueur et la première longueur est d'environ 2:1.

11. Article absorbant selon l'une quelconque des revendications précédentes, dans lequel la zone de faible allongement (402) comprend une région de liaison de ruban jetable (401), dans lequel la zone de faible allongement (402) comprend une première concentration de ligne de coupe et la région de liaison de ruban jetable (401) comprend une seconde concentration de ligne de coupe, dans lequel la seconde concentration de ligne de coupe est d'environ 30 % à environ 60 % de la première concentration de ligne de coupe.

12. Article absorbant selon la revendication 11, dans lequel la concentration de ligne de coupe de la région de liaison de ruban jetable (401) est ajustée par des lames d'amincissement, dans lequel les lames destinées à fournir la région de liaison de ruban jetable (401) sont alignées dans une même direction que les lames destinées à fournir la zone de faible allongement (402).

13. Article absorbant selon la revendication 12, dans lequel la distance entre l'une quelconque des lignes de coupe (404) dans la région de liaison de ruban jetable (401) n'est pas supérieure à environ 8 mm.

14. Article absorbant selon la revendication 11, comprenant une bande jetable (700) reliée à la région de ceinture avant (116) ou à la région de ceinture arrière (118), dans lequel au moins une partie de la bande jetable (700) chevauche la région de liaison de bande jetable (401).

15. Article absorbant selon la revendication 14, dans lequel le ruban jetable (701) comprend une extrémité distale (702), dans lequel l'extrémité distale (702) chevauche une zone de fort allongement (400).

Figure 1

Figure 2A

Figure 2B

EP 4 433 010 B1

Figure 2C

Figure 2D

Figure 2E

Figure 3

EP 4 433 010 B1

Figure 4A

Figure 4B

Figure 5

EP 4 433 010 B1

Figure 6

Figure 7

Figure 8

Figure 9

Figure 10

Figure 11

Figure 11A

EP 4 433 010 B1

Figure 12A

Figure 12B

FIG. 12C

FIG. 12D

FIG. 12E

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021126150 A1
- US 6120487 A
- US 5167897 A
- US 5360420 A
- US 5599335 A
- US 5643588 A
- US 5674216 A
- US 5702551 A
- US 5968025 A
- US 6107537 A
- US 6118041 A
- US 6153209 A
- US 6410129 B
- US 6426444 B
- US 6586652 B
- US 6627787 B
- US 6617016 B
- US 6825393 B
- US 6861571 B
- US 20130072887 A1
- US 20130211356 A1
- US 20130306226 A1
- US 4940464 A
- US 5092861 A
- US 5246433 A
- US 5569234 A
- US 5897545 A
- US 5957908 A
- US 6120489 A
- US 7569039 B
- US 20030233082 A1
- US 20050107764 A1
- US 20120061016 A1
- US 20120061015 A1
- US 20130255861 A1
- US 20130255862 A1
- US 20130255863 A1
- US 20130255864 A1
- US 20130255865 A1
- US 5628097 A
- US 5916661 A
- US 6545197 B
- US 6107539 A
- US 4610678 A
- US 4673402 A
- US 4888231 A
- US 4834735 A
- US 5562646 A
- US 5669894 A

- US 6790798 B
- US 20040158212 A1
- US 20040097895 A1
- US 3860003 A
- US 4909803 A
- US 4695278 A
- US 4795454 A
- US 4704115 A
- US 20090312730 A1
- US 63111790
- CN 2021077843
- US 20180168878 A1
- US 20180168877 A1
- US 20180168880 A1
- US 20180170027 A1
- US 20180169964 A1
- US 20180168879 A1
- US 20180170026 A1
- US 20180168889 A1
- US 20180168874 A1
- US 20180168875 A1
- US 20180168890 A1
- US 20180168887 A1
- US 20180168892 A1
- US 20180168876 A1
- US 20180168891 A1
- US 20190070042 A1
- US 20190070041 A1
- WO 20180168889 A1
- WO 20180168874 A1
- WO 20180168875 A1
- WO 20180168890 A1
- WO 20180168887 A1
- WO 20180168892 A1
- WO 20180168876 A1
- WO 20180168891 A1
- WO 20190298586 A1
- WO 20190070042 A1
- WO 20180168878 A1
- WO 20180168877 A1
- WO 20180168880 A1
- WO 20180170027 A1
- WO 20180169964 A1
- WO 20180168879 A1
- WO 20180170026 A1
- WO 20190070041 A1
- US 5681302 A
- US 6676054 B
- US 7878447 B

- US 7905446 B
- US 9156648 B
- US 4525905 A
- US 5060881 A
- US 5775380 A
- US 18947621
- US 20040219854 A1
- US 8186296 B
- US 9265672 B
- US 9248054 B
- US 9295590 B
- US 20140148773 A1
- US 3113225 A
- US 3562041 A
- US 3733238 A
- US 5110403 A
- US 6036796 A
- US 6508641 B
- US 6645330 B
- US 4854984 A
- US 6291039 B
- US 6248195 B
- US 8778127 B
- US 9005392 B
- US 20140377513 A1
- US 20140377506 A1
- US 20060094319 A1
- US 20060032578 A1
- US 20020009940 A1
- US 7867208 B
- EP 3716929 B
- US 20070219521 A1
- US 20070219521
- US 8703450 B
- US 9630901 B
- US 9822197 B
- US 20110139657
- US 20110139658 A
- US 20110152812 A
- US 20160206774 A
- US 9169366 B
- US 20190192723 A